# EUROPÄISCHE PATENTSCHRIFT

(11) **EP 3 403 716 B1**
(45) Veröffentlichungstag und Bekanntmachung des Hinweises auf die Patenterteilung: **03.03.2021**
(21) Anmeldenummer: 18170748.0
(22) Anmeldetag: 04.05.2018
(51) Int. Cl.: B01F 13/00, B01F 15/02, A61B 17/88

(54) **KNOCHENZEMENTAPPLIKATOR MIT HOHLZYLINDER AM AUSTRAGSKOLBEN**
BONE CEMENT APPLICATOR WITH HOLLOW CYLINDER AT THE APPLICATION PISTON
APPLICATEUR DE CIMENT OSSEUX DOTÉ DU CYLINDRE CREUX AU NIVEAU DU PISTON DE DISTRIBUTION

(30) Priorität: 17.05.2017 DE 102017110732
(43) Veröffentlichungstag der Anmeldung: 21.11.2018
(73) Patentinhaber: Heraeus Medical GmbH, 61273 Wehrheim (DE)
(72) Erfinder: Vogt, Sebastian, 99092 Erfurt (DE); Kluge, Thomas, 56179 Vallendar (DE); Strathausen, Rainer, 61381 Friedrichsdorf (DE)
(74) Vertreter: Schultheiss & Sterzel Patentanwälte PartG mbB

(56) Entgegenhaltungen:
- EP-A1- 2 457 531
- WO-A1-2011/089480
- WO-A1-2014/205063
- DE-A1- 2 838 222
- US-A- 3 739 947
- US-A- 4 973 168
- US-A1- 2011 056 984

## Beschreibung

Die Erfindung betrifft eine Vorrichtung zum Herstellen eines Knochenzementteigs aus einer Monomerflüssigkeit und einem Zementpulver als Ausgangskomponenten des Knochenzementteigs und zum Austragen des gemischten Knochenzementteigs.

Die Erfindung betrifft auch ein Verfahren zur Herstellung eines Knochenzementteigs, insbesondere eines pastenförmigen Polymethylmethacrylat-Knochenzementteigs, mit einer solchen Vorrichtung.

Gegenstand der Erfindung ist insbesondere eine Vorrichtung zum separaten Lagern des Zementpulvers und der Monomerflüssigkeit von Polymethylmethacrylat-Knochenzement, zum anschließenden Vermischen des Zementpulvers mit der Monomerflüssigkeit zur Bildung eines Knochenzementteigs und zum Austragen des gemischten Knochenzementteigs. Es handelt sich bei der erfindungsgemäßen Vorrichtung bevorzugt um ein Full-Prepacked-Zementiersystem.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Konventionelle Polymethylmethacrylat-Knochenzemente (PMMA-Knochenzemente) sind aus einer pulverförmigen Komponente und einer flüssigen Monomerkomponente zusammengesetzt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Die Monomerkomponente enthält im Allgemeinen das Monomer Methylmethacrylat und einen darin gelösten Aktivator (N,N-Dimethyl-p-toluidin). Die Pulverkomponente, auch als Zementpulver oder Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement beziehungsweise Knochenzementteig. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Knochenzementteigs, bis dieser erstarrt. PMMA-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1, US 5 344 232 A.

Eine Weiterentwicklung in der Zementiertechnik stellen Zementiersysteme dar, in denen sowohl das Zementpulver als auch die Monomerflüssigkeit bereits in separaten Kompartimenten der Mischvorrichtungen verpackt sind und die erst unmittelbar vor der Zementapplikation im Zementiersystem miteinander vermischt werden. Solche geschlossenen Full-Prepacked-Mischvorrichtungen wurden mit der EP 0 692 229 A1, der DE 10 2009 031 178 B3, der US 5 997 544 A, der US 6 709 149 B1, der DE 698 12 726 T2, der EP 0 796 653 A2 und der US 5 588 745 A vorgeschlagen.

Das Patent DE 10 2009 031 178 B3 offenbart eine Lager- und Mischvorrichtung als Full-Prepacked-Zementiersystem, in dem die zur Herstellung des Knochenzementteigs notwendigen Ausgangskomponenten bereits in der Lager- und Mischvorrichtung gelagert sind und in der Lager- und Mischvorrichtung zusammengeführt und gemischt werden können. Die Lager- und Mischvorrichtung weist einen zweiteiligen Austragskolben zum Verschluss einer Zementkartusche auf. Dabei wird eine Kombination aus einem gasdurchlässigen Sterilisationskolben und einem gasundurchlässigen Dichtungskolben verwendet.

Polymethylmethacrylat-Knochenzemente werden nach Vermischung des Zementpulvers mit der flüssigen Monomerkomponente im noch nicht ausgehärteten, pastenförmigen Zustand als Knochenzementteig appliziert. Bei Verwendung von Mischvorrichtungen befindet sich der Knochenzementteig im Fall von Pulver-Flüssigkeits-Zementen in einer Kartusche. Bei der Applikation solcher konventioneller PMMA-Knochenzemente, wird nach der Vermischung der beiden Ausgangskomponenten der gebildete Knochenzementteig mit Hilfe von manuell bedienbaren Auspressvorrichtungen ausgepresst. Aus der Kartusche wird der Knochenzementteig durch Bewegung eines Austragskolbens herausgedrückt. Die Austragskolben haben üblicherweise einen Durchmesser zwischen 30 mm bis 40 mm und damit an der Außenseite, an welcher ein Stößel beziehungsweise eine Stange der Auspressvorrichtung beim Auspressvorgang angreift, eine Fläche von 7,0 cm² bis 12,5 cm². Die Bewegung des Austragskolbens wird bevorzugt durch manuell bedienbare, mechanische Auspressvorrichtungen bewirkt. Diese manuellen Auspressvorrichtungen erreichen normalerweise eine Auspresskraft im Bereich von ungefähr 1,5 kN bis 3,5 N.

Diese einfachen mechanischen Auspressvorrichtungen nutzen insbesondere Klemmstangen zum Auspressen, die durch einen manuell zu betätigenden Kipphebel angetrieben werden. Die manuell angetriebenen Auspressvorrichtungen sind seit Jahrzehnten weltweit erprobt und stellen bisher den Stand der Technik dar. Vorteilhaft an diesen Auspressvorrichtungen ist, dass der medizinische Anwender über die aufzubringende Handkraft ein Gefühl für den Eindringwiderstand des Knochenzementteigs in die Knochenstrukturen (Spongiosa) hat.

Bei der Verwendung aller bisher bekannten Full-Prepacked-Zementiersysteme muss der medizinische Anwender mehrere Arbeitsschritte in einer vorbestimmten Reihenfolge an den Vorrichtungen nacheinander durchführen bis der gemischte Knochenzementteig vorliegt und appliziert werden kann. Eine Verwechslung der Arbeitsschritte kann zum Versagen der Mischvorrichtung führen und daher Störungen im OP-Ablauf verursachen. Kostenintensive Schulungen der medizinischen Anwender sind deshalb notwendig, um Anwenderfehler zu vermeiden.

Die US 2011/056984 A1 offenbart eine Vorrichtung zum Mischen eines Dentalklebstoffs, bei dem eine Monomerflüssigkeit nach Aufbrechen eines dünnen Teils einer Trennwand in ein Zementpulver gepresst wird.

In der WO 00/35506 A1 wird eine Vorrichtung vorgeschlagen, bei der Polymethylmethacrylat-Zementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpulverpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Konventionelle Zementpulver zeigen zudem das Phänomen, dass auf Grund der unterschiedlichen Oberflächenenergien die Zementpulverpartikel durch Methylmethacrylat nur schlecht benetzt werden. Dadurch dringt das Methylmethacrylat nur relativ langsam in das Zementpulver ein. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzementteigs. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren.

Aus dem Kleb- und Dichtstoffbereich sind auch elektrisch angetriebene Auspressvorrichtungen bekannt. Diese Vorrichtungen können sowohl mit Akkumulatoren und mit Batterien als auch mit Hilfe einer stationären Stromversorgung angetrieben werden. Diese Vorrichtungen können mit ihren zum Teil sehr großen Auspresskräften besonders zähe pastenförmige Massen auspressen. Nachteilig ist jedoch bei der Verwendung von Elektromotoren, dass diese Buntmetalle enthalten und kostenintensiv in der Beschaffung sind. Im steril zu haltenden OP-Bereich müssen derartige Vorrichtungen aufwendig sterilisiert oder sogar ausgetauscht werden. Bei einer elektrischen Verkabelung kann die Bewegung des Anwenders im OP behindert werden.

Weiterhin wurden auch pneumatische Vorrichtungen vorgeschlagen. Diese Geräte erfordern einen stationären oder mobilen Druckluftanschluss (US 2 446 501 A, DE 20 2005 010 206 U1). Dazu sind Druckluftschläuche notwendig, welche die Bewegung des Anwenders behindern können.

Alternativ dazu ist auch die Verwendung von Druckgaspatronen zur Bereitstellung von Druckgas möglich. Dazu wurden Vorrichtungen vorgeschlagen, bei denen der Druckgaszufluss durch ein Ventil und zusätzlich durch ein zweites Ventil der Strom der viskosen Masse gesteuert werden (US 2004/0074927 A1, US 6 935 541 B1). Bei diesen Vorrichtungen sind die Gaspatronen in den Vorrichtungen integriert. Bei derartigen an Druckluft angeschlossenen oder Druckgaspatronen enthaltenden Systemen ist immer eine Druckgasquelle erforderlich, ohne die die Systeme nicht mehr anwendbar sind.

In der nicht vorveröffentlichten DE 10 2016 121 607 wird ein Full-Prepacked-Zementiersystem mit einer Kartusche enthaltend ein Knochenzementpulver vorgeschlagen. In der Kartusche ist ein Austragskolben vorgesehen und hinter der Kartusche eine Aufnahme enthaltend einen Monomerflüssigkeitsbehälter angeordnet. An der Rückseite der Aufnahme befindet sich ein Förderkolben, mit dem der Monomerflüssigkeitsbehälter zerquetscht werden kann und die Monomerflüssigkeit aus der Aufnahme in die Kartusche gepresst werden kann.

Es zeigte sich in praktischen Versuchen, dass der mit dieser Vorrichtung hergestellte Knochenzementteig bei der Verwendung eines geeigneten Zementpulvers und eines geeigneten Gewichtsverhältnisses von Zementpulver zu Monomerflüssigkeit immer eine gute Konsistenz besitzt. Wird der geborstene Monomerflüssigkeitsbehälter beim Monomertransfer maximal komprimiert, dann erhält man reproduzierbar einen guten Zementteig. Es kann jedoch bei bestimmten Konstellationen zu einer ungewünschten Veränderung der Konsistenz des Knochenzementteigs am Ende des Auspressvorgangs kommen, bei dem sich das Mischungsverhältnis zwischen dem Zementpulver und der Monomerflüssigkeit verändert hat. Es können jedoch mitunter auch ein bis wenige kleine Monomerblasen im Volumenbereich von wenigen Mikrolitern am Rand des ausgepressten Zementteigs auftreten.

Im Rahmen der vorliegenden Erfindung wurde gefunden, dass dies mit der Wahl und der Stabilität des Monomerflüssigkeitsbehälters sowie mit dem Vordringen der Monomerflüssigkeit zwischen dem Zementpulver und der Innenwand der Kartusche zusammenhängt. Bei unvollständiger Kompression des geborstenen Monomerflüssigkeitsbehälters, die zum Beispiel durch die Wahl eines Monomerflüssigkeitsbehälters mit sehr stabilen Wandungen auftreten kann, kann nämlich ein Rest der Monomerflüssigkeit zwischen dem Austragskolben und dem Förderkolben innerhalb der Fragmente des geborstenen Monomerflüssigkeitsbehälters verbleiben, der am Ende des Auspressens des Knochenzementteigs durch eine anschließende Nachkompression des geborstenen Monomerflüssigkeitsbehälters infolge einer axialen Bewegung des Förderkolbens in Richtung des Austragskolbens durch das Austragsrohr austreten kann. Die Monomerflüssigkeit kann zwischen dem Zementpulver und der Innenwand der Kartusche entlang der Innenwand der Kartusche kriechen, ohne dass sie sich dabei zügig mit umgebenden Zementpulver durchmischt.

Die Aufgabe der vorliegenden Erfindung besteht also darin, die Nachteile des Stands der Technik zu überwinden. Insbesondere besteht die Aufgabe der Erfindung in der Entwicklung einer Vorrichtung die zum Mischen des Knochenzementteigs aus den Ausgangskomponenten und zum Austragen des gemischten Knochenzementteigs vorgesehen und geeignet ist sowie eines Verfahrens zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit mit einer solchen Vorrichtung hergestellt wird mit dem die Nachteile der bisherigen Vorrichtungen und Verfahren überwunden werden. Die Erfindung hat die Aufgabe die Bildung von Monomerblasen im erzeugten Knochenzementteig zu verhindern. Ferner hat die vorliegende Erfindung die Aufgabe, eine solche Vorrichtung derart zu verbessern, dass auch bei einer nicht vollständigen Kompression des Monomerflüssigkeitsbehälters am Ende des Austrags des Knochenzementteigs ein Austritt der Monomerflüssigkeit aus dem Austragsrohr der Kartusche wirksam verhindert wird. Mit der erfindungsgemäßen Vorrichtung und dem erfindungsgemäßen Verfahren soll also erreicht werden, dass auch bei einem sehr einfachen und kostengünstigen Aufbau der Vorrichtung und bei gleichzeitig sehr einfacher und unkomplizierter Anwendbarkeit der Vorrichtung von Anfang bis Ende des Auspressvorgangs ein homogener Knochenzementteig erzeugt und appliziert werden kann. Es ist daher Aufgabe, die in der nicht vorveröffentlichten DE 10 2016 121 607 beschriebene Vorrichtung so zu verbessern, dass die Bildung des Knochenzementteigs reproduzierbar möglich ist, ohne dass es zum stochastischen Auftreten von kleinen Monomereinschlüssen im Knochenzementteig kommt.

Die Vorrichtung soll durch eine einfache Auspressvorrichtung anzutreiben sein und dabei möglichst einfach in der Bedienung sein. Der Aufbau soll kostengünstig sein, damit die Vorrichtung aus hygienischen Gründen nur einmalig verwendet werden kann. Es sollen möglichst viele oder alle in der Vorrichtung ablaufenden Prozesse, wie die Vermischung der Ausgangskomponenten, das Austragen des Knochenzementteigs und gegebenenfalls auch das Öffnen des Monomerflüssigkeitsbehälters und gegebenenfalls auch das Öffnen der Kartusche mit möglichst wenigen Arbeitsschritten und so weit als möglich automatisiert ablaufen und vorzugsweise mit einem einzigen linearen Antrieb anzutreiben sein.

Die Aufgabe der Erfindung besteht also auch in der Entwicklung einer Vorrichtung zum Vermischen von Zementpulver und Monomerflüssigkeit. Die Handhabung der Vorrichtung soll maximal vereinfacht sein, um Anwendungsfehler infolge von fehlerhaft durchgeführten Montageschritten grundsätzlich zu vermeiden. Der medizinische Anwender soll die Vorrichtung nach der Entnahme aus einer Verpackung mit einer Auspressvorrichtung verbinden und diese anschließend betätigen. Weitere Montage- und Arbeitsschritte sollen durch den Aufbau der Vorrichtung vermieden werden. Die Vorrichtung soll vorzugsweise auch eine sichere Lagerung von Zementpulver und Monomerflüssigkeit in voneinander getrennten Kompartimenten ermöglichen, so dass während der Lagerung der Vorrichtung eine unbeabsichtigte Vermischung der Zementkomponenten ausgeschlossen ist. Die Vorrichtung soll eine Sterilisation mit dem Gas Ethylenoxid ermöglichen. Das in der Vorrichtung gelagerte Zementpulver muss dazu für Ethylenoxid zugänglich sein. Die Vorrichtung soll mit Hilfe einer im OP manuell angetriebenen Auspressvorrichtung aktivierbar sein, so dass nach der form- oder kraftschlüssigen Verbindung der Vorrichtung mit der Auspressvorrichtung durch Betätigung der Auspressvorrichtung die axial vortreibbare Stange der Auspressvorrichtung auf die Vorrichtung einwirkt, gegebenenfalls den Monomerflüssigkeitsbehälter öffnet und anschließend bei weiterer Bewegung der Stange die Monomerflüssigkeit in das Zementpulver transferiert. Die Vermischung der Monomerflüssigkeit mit dem Zementpulver soll ohne einen von außen manuell zu bewegenden Mischer erfolgen. Es soll möglichst nur mit der Vorwärtsbewegung der Stange der Auspressvorrichtung die Vermischung der Zementkomponenten unter Bildung des Knochenzementteigs und das Auspressen des gemischten Knochenzementteigs vorgenommen werden. Bevorzugt soll möglichst nur mit der Vorwärtsbewegung der Stange der Auspressvorrichtung auch das Öffnen des Monomerflüssigkeitsbehälters und der nachfolgende Monomerflüssigkeitstransfer in das Zementpulver vorgenommen werden.

Die Aufgaben der Erfindung werden gelöst durch eine Vorrichtung zum Herstellen eines Knochenzementteigs aus einer Monomerflüssigkeit und einem Zementpulver als Ausgangskomponenten des Knochenzementteigs und zum Austragen des gemischten Knochenzementteigs, die Vorrichtung aufweisend eine Kartusche mit einem zylindrischen Innenraum, wobei der Innenraum der Kartusche an der Vorderseite bis auf eine Austragsöffnung zum Austreiben des Knochenzementteigs verschlossen ist, wobei im Innenraum der Kartusche ein in Richtung der Austragsöffnung drückbarer Austragskolben angeordnet ist und wobei das Zementpulver im Innenraum der Kartusche zwischen der Austragsöffnung und dem Austragskolben angeordnet ist, wobei an der der Austragsöffnung zugewandten Vorderseite des Austragskolbens ein Hohlzylinder angeordnet ist, wobei der Hohlzylinder an seiner der Austragsöffnung zugewandten Vorderseite offen ist und sich von der Vorderseite des Austragskolbens zumindest 3 mm in den Innenraum der Kartusche erstreckt, wobei in dem Austragskolben zumindest eine Verbindung von der Rückseite des Austragskolbens zur Vorderseite des Austragskolbens zum Einleiten der Monomerflüssigkeit in den Innenraum der Kartusche vorgesehen ist, wobei die zumindest eine Verbindung für die Monomerflüssigkeit und Gase durchlässig ist und für das Zementpulver undurchlässig ist und wobei die zumindest eine Verbindung aus dem Austragskolben innerhalb des Hohlzylinders oder durch Leitungen in dem Hohlzylinder an der Vorderseite des Hohlzylinders in den Innenraum der Kartusche münden.

Es kann bevorzugt vorgesehen sein, dass der Austragskolben gegen die Innenwand des Innenraums der Kartusche dicht ist oder abgedichtet ist, insbesondere mit zumindest einer umlaufenden Dichtung abgedichtet ist.

Bevorzugt ist die erfindungsgemäße Vorrichtung auch zum Lagern des Zementpulvers vorgesehen und besonders bevorzugt auch zum Lagern der Monomerflüssigkeit vorgesehen.

Es kann vorgesehen sein, dass die Austragsöffnung in der Vorderseite der Kartusche angeordnet ist. Bevorzugt wird oder ist an der Vorderseite der Kartusche ein Austragsrohr angeordnet, dass die Austragsöffnung begrenzt.

Der Hohlzylinder ist im Innenraum der Kartusche angeordnet.

Dass sich der Hohlzylinder von dem Austragskolben in Richtung der Vorderseite der Kartusche und damit im Innenraum der Kartusche erstreckt, bedeutet, dass ein Totvolumen von dem Hohlzylinder im Innenraum der Kartusche begrenzt wird. Dadurch, dass ein Totvolumen im Innenraum der Kartusche verbleibt, kann zwischen der Austragsöffnung und dem Austragskolben ein Volumen verbleiben, das mit einer Mischung aus dem Zementpulver und der Monomerflüssigkeit gefüllt ist, wenn der Hohlzylinder gegen die Vorderseite des Innenraums der Kartusche gedrückt ist und der Austragskolben dadurch nicht weiter in Richtung der Austragsöffnung vorgetrieben werden kann.

Die Kartusche, der Austragskolben und der Hohlzylinder sind vorzugsweise aus einem thermoplastischen Kunststoff gefertigt, insbesondere mit einem Spritzgussverfahren.

Der Innenraum der Kartusche hat eine zylindrische Geometrie. Die zylindrische Form ist die einfachste, mit der sich der Innenraum der Kartusche realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die Innenwand des Innenraums der Kartusche kann also durch den Zylindermantel eines Zylinders mit beliebiger Grundfläche realisiert sein, insbesondere mit unterschiedlicher Grundfläche realisiert sein, das heißt auch mit nicht kreisförmigen oder nicht runden Grundflächen. Erfindungsgemäß wird jedoch eine zylindrische Geometrie mit rotationssymmetrischer und insbesondere kreisrunder Grundfläche für den Innenraum bevorzugt, da diese am einfachsten zu fertigen ist.

Bevorzugt ist die Vorderseite des Austragskolbens bis auf den Hohlzylinder eben.

Bei erfindungsgemäßen Vorrichtungen kann vorgesehen sein, dass der Hohlzylinder an seiner äußeren Mantelfläche maximal 0,5 mm von der Innenwand des Innenraums der Kartusche beabstandet ist, bevorzugt maximal 0,1 mm von der Innenwand des Innenraums der Kartusche beabstandet ist.

Hierdurch wird sichergestellt, dass sich zwischen der Innenwand des Innenraums der Kartusche und der äußeren Mantelfläche des Hohlzylinders kein oder nur wenig Zementpulver befindet, dass für die Monomerflüssigkeit nur schwer zu erreichen ist und das die Bewegung des Austragskolbens behindern würde.

Es kann auch vorgesehen sein, dass der Hohlzylinder zumindest bereichsweise an der Innenwand des Innenraums der Kartusche anliegt, vorzugsweise an seiner äußeren Mantelfläche an der Innenwand des Innenraums der Kartusche anliegt.

Damit wird sichergestellt, dass sich zwischen der Innenwand des Innenraums der Kartusche und der äußeren Mantelfläche des Hohlzylinders kein Zementpulver befindet, dass für die Monomerflüssigkeit nur schwer zu erreichen ist und das die Bewegung des Austragskolbens behindern würde.

Des Weiteren ist vorgesehen, dass der Hohlzylinder eine weitere Bewegung des Austragskolbens in Richtung der Vorderseite der Kartusche blockiert, wenn die Vorderseite des Hohlzylinders an der Vorderseite des Innenraums der Kartusche anliegt, so dass der Austragskolben von der Vorderseite des Innenraums der Kartusche beabstandet ist und ein Totvolumen in dem Innenraum der Kartusche verbleibt.

Damit wird erreicht, dass das in dem Hohlzylinder eingeschlossene Totvolumen einen Rest des erzeugten Knochenzementteigs, der schlechter gemischt ist oder der sich aufgrund von in den Innenraum der Kartusche nachströmender Monomerflüssigkeit am Ende des Auspressvorgangs eine sich ändernde Konsistenz aufweist, in der Kartusche zurückhält. Ferner kann vorgesehen sein, dass der Hohlzylinder zumindest einen Schlitz aufweist, vorzugsweise zumindest einen parallel zur Zylinderachse des Hohlzylinders verlaufenden Schlitz aufweist, besonders bevorzugt zumindest einen von der Vorderseite bis zum Austragskolben reichenden Schlitz aufweist.

Hierdurch kann die Passung des Hohlzylinders an die Innenwand der Kartusche leichter angepasst werden und die Gefahr einer Blockade der Bewegung des Austragskolbens mit dem Hohlzylinder wird reduziert. Der zumindest Schlitz kann alternativ zu einem Verlauf parallel zur Zylinderachse des Hohlzylinders auch Spiralförmig in der Wandung des Hohlzylinders verlaufen.

Es ist vorgesehen, dass in dem Austragskolben zumindest eine Verbindung von der Rückseite des Austragskolbens zur Vorderseite des Austragskolbens zum Einleiten der Monomerflüssigkeit in den Innenraum der Kartusche vorgesehen ist, wobei die zumindest eine Verbindung für die Monomerflüssigkeit und Gase durchlässig ist und für das Zementpulver undurchlässig ist und wobei die zumindest eine Verbindung aus dem Austragskolben innerhalb des Hohlzylinders oder durch Leitungen in dem Hohlzylinder an der Vorderseite des Hohlzylinders in den Innenraum der Kartusche münden.

Dadurch muss die Monomerflüssigkeit, wenn Sie durch die Durchführung und im Inneren des Hohlzylinders in das Zementpulver geleitet wird zunächst durch das Zementpulver im Inneren des Hohlzylinders fließen und kann nicht an der Innenwand der Kartusche am Zementpulver vorbei strömen und so bis zur Austragsöffnung gelangen. Wenn die Monomerflüssigkeit durch die Leitungen im Hohlzylinder in den Innenraum der Kartusche geleitet wird, strömt diese in einem dichter zu Mitte des Innenraums der Kartusche gelegenen Bereich, so dass sich die Monomerflüssigkeit von dort auch in Richtung des Austragskolbens ausbreiten kann und sich dabei besser verteilt. Bevorzugt ist die Einmündung der Leitungen in den Innenraum der Kartusche im Bereich des inneren Mantels des Hohlzylinders gelagert. Dadurch wird sichergestellt, dass die Monomerflüssigkeit nicht auf kürzestem Weg bis zur Innenwand des Innenraums der Kartusche strömen kann. Alle diese Maßnahmen dienen dazu, dass der erzeugte Knochenzementteig und der aus der Vorrichtung ausgetragene Knochenzementteig eine bessere Homogenität aufweist und sich keine oder möglichst wenige Einschlüsse der Monomerflüssigkeit in dem Knochenzementteig bilden.

Mit einer Weiterbildung der vorliegenden Erfindung wird vorgeschlagen, dass in dem von dem Hohlzylinder umschlossenen Teil des Innenraums der Kartusche Zementpulver enthalten ist, insbesondere eingepresst ist.

Hiermit wird klargestellt, dass in dem von Hohlzylinder umschlossenen Teil ein Totvolumen für den Knochenzementteig gebildet werden soll und dass sich darin nichts Anderes befindet.

Bevorzugt kann ferner vorgesehen sein, dass das von dem Hohlzylinder begrenzte Volumen des Innenraums der Kartusche zumindest 1 cm³ groß ist, bevorzugt zumindest 3 cm³ groß ist.

Hiermit wird sichergestellt, dass das eingeschlossene Totvolumen ausreichend groß ist, um die am Ende des Mischvorgangs entstehende Restmenge von sich in der Konsistenz ändernden Knochenzementteig aufzunehmen, ohne dass dieser mit der Vorrichtung ausgetragen und appliziert werden kann. Diese Totvolumina sind ausreichend, um nicht vollständig gemischte Anteile des Knochenzementteigs, die im Bereich des Austragskolbens in dem Innenraum der Kartusche entstehen können, im Innenraum der Kartusche zurückzuhalten. Dadurch kann verhindert werden, dass am Ende des Austragsvorgangs schlecht gemischter Knochenzementteig oder ein Knochenzementteig mit sich ändernder Zusammensetzung und damit Konsistenz ausgetragen wird, der nicht verwendbar ist.

Gemäß einer bevorzugten Weiterbildung der vorliegenden Erfindung kann vorgesehen sein, dass der Hohlzylinder sich von der Vorderseite des Austragskolbens zumindest 5 mm in den Innenraum der Kartusche erstreckt, bevorzugt zumindest 7,5 mm in den Innenraum der Kartusche erstreckt, besonders bevorzugt zumindest 10 mm in den Innenraum der Kartusche erstreckt.

Damit wird einerseits das Totvolumen in dem vom Hohlzylinder eingeschlossenen Bereich vergrößert und andererseits der Weg bis zur Grenzfläche zwischen dem Zementpulver und der Innenwand der Kartusche vergrößert, den die Monomerflüssigkeit durch das Zementpulver zurücklegen muss, bevor die Gefahr besteht, dass die Monomerflüssigkeit an dem Zementpulver oder an bereits entstandenem Knochenzementteig vorbei entlang der Innenwand der Kartusche strömen kann.

Ferner kann vorgesehen sein, dass die Wandstärke des Hohlzylinders zumindest 1 mm beträgt, bevorzugt zumindest 1,5 mm beträgt, besonders bevorzugt zumindest 2 mm beträgt. Auch diese Maßnahme dient dazu den Weg der Monomerflüssigkeit bis zur Innenwand der Kartusche zu verlängern und dadurch eine größere Homogenität des erzeugten Knochenzementteigs zu erreichen. Zudem wird so eine ausreichende Stabilität des vorzugsweise aus Kunststoff bestehenden Hohlzylinders bewirkt, so dass dieser am Ende des Auspressvorgangs nicht verformt oder nicht zu stark verformt wird.

Des Weiteren kann mit einer besonders bevorzugten Form der vorliegenden Erfindung, die auch zum Lagern der Monomerflüssigkeit geeignet ist und somit ein Full-Prepacked-System bereitstellt, vorgesehen sein, dass die Vorrichtung eine Aufnahme aufweist, in der die Monomerflüssigkeit, insbesondere ein Monomerflüssigkeitsbehälter enthaltend die Monomerflüssigkeit, enthalten ist, wobei die Rückseite der Kartusche mit der Vorderseite der Aufnahme verbunden ist, vorzugsweise derart verbunden ist, dass der Innenraum der Kartusche mit dem Innenraum der Aufnahme fluchtet.

Hierdurch ist die Vorrichtung auch zum Lagern der Monomerflüssigkeit geeignet sowie zum Mischen der Monomerflüssigkeit mit dem Zementpulver innerhalb der Vorrichtung. Die Vorrichtung ist damit ein Full-Prepacked-Zementiersystem. Durch die fluchtenden Innenräume der Kartusche und der Aufnahme kann sichergestellt werden, dass zunächst der Förderkolben durch einen auf die Rückseite des Förderkolbens wirkenden Druck bewegt werden kann und anschließend der Förderkolben zum Antreiben des Austragskolbens genutzt werden kann, indem der Förderkolben gemeinsam mit dem Austragskolben weiter in Richtung der Austragsöffnung gedrückt wird.

Die Aufnahme ist vorzugsweise aus einem thermoplastischen Kunststoff gefertigt, insbesondere mit einem Spritzgussverfahren.

Hierdurch kann die Vorrichtung kostengünstig als hygienisches Wegwerfprodukt gefertigt werden.

Bei erfindungsgemäßen Vorrichtungen, bei denen die Monomerflüssigkeit in einem Monomerflüssigkeitsbehälter innerhalb der Vorrichtung angeordnet ist, kann vorgesehen sein, dass der Monomerflüssigkeitsbehälter eine Glasampulle, eine Plastikampulle, ein Kunststofffolienbeutel oder ein Aluminium-Kunststoff-Verbund-Beutel ist. In derartigen Monomerflüssigkeitsbehältern kann die Monomerflüssigkeit besonders lange gelagert werden.

Bei Vorrichtungen mit Aufnahme kann auch vorgesehen sein, dass ein Innenraum der Aufnahme und der Innenraum der Kartusche über eine für die Monomerflüssigkeit und für Gase durchlässige aber für das Zementpulver undurchlässige Verbindung miteinander verbunden sind.

Hiermit wird gestellt, dass das Zementpulver nicht durch die Verbindung in den Innenraum der Aufnahme vordringt, dort vorzeitig mit der Monomerflüssigkeit reagiert und anschließend den Monomertransfer in den Innenraum der Kartusche verhindert. Die Verbindung ist besonders bevorzugt im Austragskolben angeordnet.

Ferner kann vorgesehen sein, dass die Aufnahme einen zylindrischen Innenraum aufweist, in dem die Monomerflüssigkeit, insbesondere ein Monomerflüssigkeitsbehälter enthaltend die Monomerflüssigkeit, angeordnet ist.

Der Innenraum der Aufnahme hat eine zylindrische Geometrie. Die zylindrische Form ist auch hier die einfachste, mit der sich der Innenraum der Aufnahme realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche.

Des Weiteren kann vorgesehen sein, dass in der Aufnahme ein in Längsrichtung der Aufnahme beweglicher Förderkolben angeordnet ist, der von einer Rückseite der Aufnahme aus in Richtung der Vorderseite vortreibbar ist, wobei die Monomerflüssigkeit, insbesondere ein Monomerflüssigkeitsbehälter enthaltend die Monomerflüssigkeit, zwischen dem Förderkolben und dem Austragskolben angeordnet ist.

Hiermit wird ein Full-Prepacked-Zementiersystem bereitgestellt, in dem alle Ausgangskomponenten des Knochenzementteigs, nämlich die Monomerflüssigkeit und das Zementpulver enthalten sind und dort auch gelagert werden können.

Der Förderkolben schließt die Aufnahme an deren Rückseite flüssigkeitsdicht ab, bis auf gegebenenfalls vorhandene Belüftungsöffnungen (siehe unten).

Es kann dabei vorgesehen sein, dass an der Vorderseite des Förderkolbens zumindest eine vortretende Spitze, Kante und/oder Schneide zum Brechen des Monomerflüssigkeitsbehälters angeordnet ist.

Durch die Anwendung einer definierten Kraft an einer vorherbestimmten und räumlich begrenzten Stelle kann der Druck an dieser Stelle bei gleicher Kraft erhöht werden und so ein definiertes Brechen des Monomerflüssigkeitsbehälters erreicht werden. Dadurch wird der Ablauf des Aufbrechens des Monomerflüssigkeitsbehälters reproduzierbarer.

Bei erfindungsgemäßen Vorrichtungen mit Förderkolben kann alternativ oder zusätzlich vorgesehen sein, dass der Monomerflüssigkeitsbehälter im Inneren der Aufnahme durch eine Bewegung des Förderkolbens in Richtung der Vorderseite der Aufnahme zu öffnen ist, bevorzugt aufzubrechen oder aufzureißen ist.

Hierdurch wird erreicht, dass durch die axiale lineare Bewegung des Förderkolbens der Monomerflüssigkeitsbehälter geöffnet werden kann. Es kann dadurch eine Auspressvorrichtung mit nur einer Stange als axialer linearen Antrieb verwendet werden, um sowohl den Monomerflüssigkeitsbehälter zu öffnen als auch die Monomerflüssigkeit in die Kartusche zu pressen und auch den Knochenzementteig aus der Kartusche zu pressen.

Mit einer Weiterbildung der vorliegenden Erfindung kann vorgesehen sein, dass in der Wandung der Aufnahme zumindest eine Belüftungsöffnung angeordnet ist, die den Innenraum der Aufnahme mit der Umgebung verbindet.

Hierdurch kann der Innenraum der Aufnahme mit einem sterilisierenden Gas sterilisiert werden.

Dabei kann vorgesehen sein, dass die zumindest eine Belüftungsöffnung derart dicht im Bereich des Förderkolbens angeordnet ist, dass sie durch eine Bewegung des Förderkolbens in Richtung der Vorderseite der Aufnahme verschlossen wird, bevor ein in der Aufnahme angeordneter Monomerflüssigkeitsbehälter, in dem die Monomerflüssigkeit enthalten ist, durch die Bewegung des Förderkolbens geöffnet ist.

Hierdurch kann die Monomerflüssigkeit nicht aus dem Innenraum der Aufnahme austreten, wenn die zumindest eine Belüftungsöffnung von dem sich in Richtung der Vorderseite der Aufnahme bewegenden Förderkolben verschlossen wird, bevor der Monomerflüssigkeitsbehälter durch die Bewegung des Förderkolbens geöffnet wird, also beispielsweise von dem Förderkolben im Innenraum der Aufnahme zerdrückt, zersplittert oder aufgerissen wird.

Bevorzugt kann vorgesehen sein, dass die Aufnahme und die Kartusche einteilig durch einen röhrenförmigen Behälter gebildet sind.

Dieser Aufbau ist der einfachste und am kostengünstigsten zu realisierende Aufbau.

Es kann auch vorgesehen sein, dass an der Rückseite der Vorrichtung, ein Befestigungsmittel zur Befestigung einer Auspressvorrichtung angeordnet ist, mit der der Austragskolben in Richtung der Austragsöffnung drückbar ist.

Hiermit kann die Vorrichtung an einer Auspressvorrichtung mit einer vortreibbaren Stange angeschlossen und befestigt werden.

Es kann vorgesehen sein, dass das Zementpulver an der Vorderseite des Austragskolbens anliegt, insbesondere vollflächig anliegt, wobei vorzugsweise das Zementpulver in den Innenraum der Kartusche eingepresst ist.

Hierdurch wird verhindert, dass in der Kartusche größere Gaseinschlüsse verbleiben, die beim Mischen der Monomerflüssigkeit mit dem Zementpulver zu Gaseinschlüssen im Knochenzementteig führen könnten. Dies kann bei einem dicht geschütteten oder vorzugsweise gepressten Zementpulver nicht passieren, da die Monomerflüssigkeit die Partikel des Zementpulvers gut benetzt und die Oberflächenspannung der Monomerflüssigkeit dann keine oder zumindest keine relevanten Gaseinschlüsse zwischen den Partikeln des Zementpulvers erlaubt.

Des Weiteren kann vorgesehen sein, dass die Austragsöffnung an deren Vorderseite mit einem Verschluss, insbesondere mit einem Stopfen, verschlossen ist, wobei der Knochenzementteig durch die Austragsöffnung aus der Kartusche auspressbar ist, wenn die Austragsöffnung geöffnet ist, und wobei vorzugsweise der Verschluss für Gase durchlässig und für das Zementpulver undurchlässig ist.

Dadurch kann die Kartusche gut zur Lagerung des Zementpulvers verwendet werden. Der Verschluss kann geöffnet werden. Der Innenraum der Kartusche und das Zementpulver können durch Evakuieren und Spülen des Innenraums der Kartusche mit einem sterilisierenden Gas, wie Ethylenoxid, durch den Verschluss hindurch sterilisiert werden, wenn dieser für Gase durchlässig und für das Zementpulver undurchlässig ist.

Der Verschluss ist vorzugsweise ein für Gase durchlässiger und für das Zementpulver undurchlässiger Filter, insbesondere Porenfilter.

Bei Vorrichtungen mit Verschluss kann vorgesehen sein, dass der Verschluss an der dem Innenraum der Kartusche zugewandten Rückseite eine Vertiefung aufweist, in der der vorderste Teil des Zementpulvers enthalten ist.

Dadurch wird erreicht, dass der vorderste Teil des Knochenzementteigs, der in der Vertiefung enthalten ist, mit dem Verschluss entfernt werden kann. In diesem Teil ist die Monomerflüssigkeit zuletzt angelangt, wenn sie von der Rückseite aus in das Zementpulver eingepresst wird. Dadurch kann also ein weniger stark durchmischter Teil des Knochenzementteigs mit dem Verschluss entfernt werden.

Der Verschluss bildet vorzugsweise mit dem Austragskolben ein durch axialen Druck auf den Austragskolben in Richtung der Austragsöffnung wirkenden Druck zu öffnendes Verschlusssystem der Kartusche.

Des Weiteren kann vorgesehen sein, dass an der Vorderseite der Kartusche ein Austragsrohr angeordnet, wobei der Knochenzementteig durch das Austragsrohr auspressbar ist.

Hiermit kann die Vorrichtung gut zur Applikation von Knochenzementteig an schwerer zugänglichen Stellen verwendet werden.

Es kann dazu auch vorgesehen sein, dass das Volumen der Zwischenräume zwischen den Zementpartikeln des Zementpulvers Innenraum der Kartusche im Bereich von 22 Volumenprozent bis 40 Volumenprozent bezogen auf das Gesamtvolumen des Zementpulvers ist. Das Gesamtvolumen des Zementpulvers entspricht bevorzugt dem Volumen des Innenraums der Kartusche, der durch den Austragskolben und durch einen Verschluss in einer Austragsöffnung an der Vorderseite der Kartusche begrenzt ist.

Bevorzugte erfindungsgemäße Vorrichtungen können sich auch dadurch auszeichnen, dass der Querschnitt des Innenraums der Kartusche maximal 16 cm² beträgt, bevorzugt maximal 5 cm² beträgt.

Es kann analog auch vorgesehen sein, dass der Innendurchmesser der Kartusche kleiner als 50 mm ist, bevorzugt kleiner als 20 mm ist.

Durch den geringen Innendurchmesser ist der Querschnitt des Innenraums der Kartusche so klein, dass der zähe Knochenzementteig aus der Kartusche mit Hilfe einer manuell angetriebenen Auspressvorrichtung ausgepresst werden kann, auch wenn noch weitere, die Strömung behindernde Leitungen, wie ein Schlauch, ein Applikatorrohr oder ein statischer Mischer in Flussrichtung des Knochenzementteigs vorgesehen sind.

Es kann ferner vorgesehen sein, dass das Volumen der Monomerflüssigkeit in der Vorrichtung, insbesondere der Monomerflüssigkeit in einem Monomerflüssigkeitsbehälter in der Vorrichtung, mindestens so groß ist wie das Volumen der mit Luft gefüllten Zwischenräume zwischen den Zementpulverpartikeln in der Kartusche, bevorzugt mindestens so groß ist wie das Volumen der Flüssigkeitsleitungen zwischen dem Innenraum der Kartusche und dem Innenraum einer Aufnahme, in der die Monomerflüssigkeit enthalten ist, plus das Volumen der mit Luft gefüllten Zwischenräume zwischen den Zementpulverpartikeln in der Kartusche. Hierdurch kann sichergestellt werden, dass das gesamte Zementpulver von der Monomerflüssigkeit benetzt werden kann und so ein homogener Knochenzementteig erzeugt wird.

Ferner kann vorgesehen sein, dass der Hohlzylinder einteilig mit dem Austragskolben oder mit einem an der Innenwand der Kartusche anliegenden Teil des Austragskolbens ausgebildet ist. Hierdurch können der Hohlzylinder und der Austragskolben als einteiliges Bauteil verwendet werden, wodurch die Kosten für die Herstellung reduziert werden. Die Teile der Vorrichtung (Kartusche, Hohlzylinder, Austragskolben) können aus Kunststoff durch Spritzgießen hergestellt werden.

Die der vorliegenden Erfindung zugrundeliegenden Aufgaben werden auch gelöst durch ein Verfahren zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit mit einer Vorrichtung nach einem der vorangehenden Ansprüche hergestellt wird, mit den folgenden nacheinander ablaufenden Schritten:
a) Einsetzen der Vorrichtung in eine Auspressvorrichtung, die Auspressvorrichtung aufweisend eine axial vortreibbare Stange,
a1) Vortreiben eines Förderkolben mit der Stange in Richtung der Kartusche der innerhalb einer an der Rückseite der Kartusche angeordneten Aufnahme an der Rückseite der Aufnahme beweglich gelagert ist, wobei durch die Bewegung des Förderkolbens ein Monomerflüssigkeitsbehälter, in dem die Monomerflüssigkeit enthalten ist, geöffnet wird und Eindrücken der Monomerflüssigkeit in den Innenraum der Kartusche durch die Bewegung des Förderkolbens, so dass sich die Monomerflüssigkeit mit dem Zementpulver im Innenraum der Kartusche mischt, wobei die Monomerflüssigkeit den Hohlzylinder umfließt, bevor sie bis zur Innenwand der Kartusche gelangt,
b) der Austragskolben wird mit der Stange in Richtung der Austragsöffnung der Kartusche vorgetrieben, wobei durch die Bewegung des Austragskolbens die Mischung aus dem Zementpulver und der Monomerflüssigkeit aus der Kartusche als Knochenzementteig aus der Vorrichtung ausgetrieben wird, und
c) die Vorderseite des Hohlzylinders auf die Vorderseite des Innenraums der Kartusche trifft, wobei mit dem Hohlzylinder eine weitere Bewegung des Austragskolbens in Richtung der Austragsöffnung blockiert wird und eine Restmenge der Mischung des Zementpulvers mit der Monomerflüssigkeit in einem von dem Hohlzylinder begrenzten Teil des Innenraums der Kartusche verbleibt.

Dabei kann vorgesehen sein, dass in Schritt a) die Monomerflüssigkeit durch zumindest eine für das Zementpulver undurchlässige aber für Gase und die Monomerflüssigkeit durchlässige Verbindung im Austragskolben in die Kartusche gepresst wird oder durch die Verbindung und zumindest eine Leitung im Hohlzylinder in die Kartusche gepresst wird, vorzugsweise durch eine Bewegung eines Förderkolbens, der mit der Stange der Auspressvorrichtung angetrieben wird, in die Kartusche gepresst wird.

Hiermit wird erreicht, dass die Flussrichtung der Monomerflüssigkeit die gleiche Richtung hat, wie die Bewegung des Austragskolbens, mit dem der Knochenzementteig aus der Kartusche ausgetrieben wird. Dies hat den Vorteil, dass ein einziger unidirektionaler Antrieb sowohl zum Einpressen der Monomerflüssigkeit als auch zum Ausdrücken des Knochenzementteigs verwendet werden kann. Dadurch sind herkömmliche Auspressvorrichtungen wie manuell angetriebene Kartuschenpistolen für das erfindungsgemäße Verfahren einsetzbar.

Ferner ist vorgesehen, dass in Schritt a) zuerst das Einsetzen der Vorrichtung in die Auspressvorrichtung erfolgt, daran anschließend ein Förderkolben, der innerhalb einer an der Rückseite der Kartusche angeordneten Aufnahme an der Rückseite der Aufnahme beweglich gelagert ist, mit der Stange in Richtung der Kartusche vorgetrieben wird, wobei durch die Bewegung des Förderkolbens ein Monomerflüssigkeitsbehälter, in dem die Monomerflüssigkeit enthalten ist, geöffnet wird und die Monomerflüssigkeit aus der Aufnahme in die Kartusche gepresst wird, wobei im Innenraum der Kartusche sich das Zementpulver mit der Monomerflüssigkeit mischt.

Hierdurch ist das Verfahren auch zur vorherigen Lagerung der Ausgangskomponenten geeignet. Dadurch kann das Verfahren jederzeit durch Anwendung eines kompakten Full-Prepacked-Zementiersystems angewendet werden.

Dabei kann vorgesehen sein, dass beim Vortreiben des Förderkolbens der zerbrochene oder aufgeschlitzte oder aufgeplatzte Monomerflüssigkeitsbehälter zusammengeschoben wird und gleichzeitig Gas aus der Aufnahme durch eine Verbindung in die Kartusche gedrückt wird und durch das Zementpulver in der Kartusche nach außen gedrückt wird.

Des Weiteren kann vorgesehen sein, dass in Schritt b) durch den auf die Mischung des Zementpulvers mit der Monomerflüssigkeit wirkenden Druck ein Verschluss, insbesondere ein Porenfilter, in einer Austragsöffnung an der Vorderseite der Kartusche bewegt oder hervorgedrückt wird, wobei bevorzugt hierauf der Verschluss aus der Austragsöffnung entfernt wird und besonders bevorzugt danach ein Applikationsrohr oder eine Austragsrohrverlängerung an der Vorderseite der Kartusche befestigt wird.

Dadurch kann verhindert werden, dass das in der Kartusche enthaltene Zementpulver aus der Kartusche rieseln kann oder das Pulver von außen verunreinigt wird. Gleichzeitig kann der Inhalt der Kartusche mit einem sterilisierenden Gas, wie Ethylenoxid, sterilisiert werden.

Der Erfindung liegt die überraschende Erkenntnis zugrunde, dass es mit dem Hohlzylinder an der Vorderseite des Austragskolbens gelingt, die Monomerflüssigkeit beim Einpressen in das Zementpulver im Innenraum der Kartusche über eine größere Strecke durch das Zementpulver fließen zu lassen beziehungsweise zu leiten, bevor die Monomerflüssigkeit die Innenwand der Kartusche erreicht, und dass dadurch die Ausbildung von Monomerflüssigkeitsblasen beziehungsweise Einschlüssen der Monomerflüssigkeit in dem gebildeten Knochenzementteig vermieden beziehungsweise reduziert werden kann. Dadurch wird ein homogenerer Knochenzementteig erzeugt. Ferner wurde gefunden, dass es durch das Zurückhalten eines kleinen Rests der in der Kartusche entstandenen Mischung des Zementpulvers mit der Monomerflüssigkeit in dem Innenraum der Kartusche gelingt, dass am Ende des Auspressvorgangs kein Knochenzementteig mit einer veränderten Konsistenz ausgetragen wird, da der restliche Knochenzementteig in der Kartusche zurückgehalten wird und die Austragsöffnung verschlossen wird.

Die bevorzugte erfindungsgemäße Vorrichtung in Ihrer Weiterbildung als Full-Prepacked-Zementiersystem hat die wesentlichen Vorteile, dass die beiden Ausgangskomponenten des Knochenzementteigs im geschlossenen Zementiersystem gelagert sind und dass die Vermischung der Ausgangskomponenten in der geschlossenen Vorrichtung erfolgt. Das bedeutet, dass die Vorrichtung nicht vom Anwender befüllt werden muss. Der medizinische Anwender hat keinerlei Kontakt mit den einzelnen Ausgangskomponenten der Knochenzemente. Die Geruchsbelästigung ist dadurch nur noch minimal. Ein besonderer Vorteil der Vorrichtung besteht auch darin, dass durch das einfache Vorwärtsbewegen einer Stange einer manuell angetriebenen Auspressvorrichtung die Monomerflüssigkeit in das Zementpulver gepresst wird. Dabei wird die zwischen den Zementpulverpartikeln vorhandene Luft durch die Monomerflüssigkeit verdrängt. Es entsteht ein homogener Knochenzementteig, ohne dass ein manuelles Mischen mit Mischstäben mit Mischflügeln notwendig ist. Das bedeutet, dass das fehlerbehaftete manuelle Mischen nicht mehr notwendig ist. Die Bedienung der Vorrichtung ist maximal vereinfacht. Es handelt sich um ein Ready-to-use-System.

Die Vorteile erfindungsgemäßer Vorrichtungen und Verfahren beruhen grundsätzlich darauf, dass die an sich bekannte lineare Vorwärtsbewegung von Stangen von manuell betriebenen Auspressvorrichtungen so genutzt werden, dass durch kontinuierliche Einwirkung der Kraft der linearen Vorwärtsbewegung der Stange zuerst ein Monomerflüssigkeitsbehälter geöffnet wird, der Monomerflüssigkeitsbehälter anschließend zusammengepresst wird, wodurch die Monomerflüssigkeit aus dem Monomerflüssigkeitsbehälter austritt und in verdichtetes Zementpulver eingepresst wird, wobei die zwischen den Zementpulverpartikeln vorhandene Luft durch die eingepresste Monomerflüssigkeit verdrängt wird und nach Benetzung der Zementpulverpartikel durch die Monomerflüssigkeit ein Knochenzementteig entsteht. Voraussetzung dafür ist die Verwendung eines Zementpulvers, das so eingestellt ist, dass es sehr gut von der Monomerflüssigkeit benetzt wird und diese durch Kapillarwirkung einsaugen kann.

Die Vorrichtung kann als hygienisches Wegwerfprodukt verwendet werden, da sie sehr weitgehend aus Kunststoff gefertigt werden kann und weil alle Teile einschließlich der Innenräume und des Zementpulvers mit Hilfe von Ethylenoxid sterilisierbar sind.

Die erfindungsgemäße Vorrichtung ist dadurch charakterisiert, dass das Auftreten von Monomerblasen im ausgepressten Zementteig wirksam unterbunden wird, wenn am Austragskolben in Richtung des Kartuschenkopfs beziehungsweise in Richtung der Vorderseite der Kartusche ein Hohlzylinder mit einer axialen Höhe von mindestens 3,0 mm und einer Wandstärke von mindestens 1,0 mm angebracht wird. Dadurch wird die Monomerflüssigkeit mindestens über eine Strecke von 4,0 mm innerhalb des Hohlzylinders in das Zementpulver eingeleitet, wenn die Monomerflüssigkeit an der Vorderseite des Austragskolbens im Inneren des Hohlzylinders in das Zementpulver im Innenraum der Kartusche eingeleitet beziehungsweise eingepresst wird. Dadurch wird während des Einpressens der Monomerflüssigkeit wirksam verhindert, dass sich Teile der Monomerflüssigkeit zwischen dem Zementpulver beziehungsweise dem gebildeten Zementteig und der Kartuscheninnenwand bewegen und Monomerblasen bilden können.

Eine beispielhafte Vorrichtung zum Lagern, Vermischen und Austragen von Polymethylmethacrylat-Knochenzement kann beispielsweise aufweisen:
a) einen hohlzylinderförmigen Behälter mit einem am Kartuschenende angeordneten Verbindungselement zur Verbindung mit einer Auspressvorrichtung,
b) einen Kartuschenkopf, der den hohlzylinderförmigen Behälter an der Vorderseite abschließt, wobei eine Durchführung zur Aufnahme des Austragsrohrs als Austragsöffnung im Kartuschenkopf angeordnet ist, und wobei mindestens eine Durchführung die Außenseite des Kartuschenkopfs mit der Innenseite des Kartuschenkopfs gasdurchlässig verbindet,
c) ein Austragsrohr,
d) einen axial im Kartuschenkopf beweglichen Verschluss, der gasdurchlässig aber für Pulverpartikel undurchlässig ist, wobei der Verschluss eine von der Unterseite zur Oberseite gehende Durchführung besitzt, die an der Oberseite mit dem Austragsrohr flüssigkeitsdurchlässig verbunden ist,
e) einen Förderkolben, der in dem Behälter axial beweglich angeordnet ist, der den Kartuschenboden flüssigkeitsundurchlässig verschließt,
f) einen Austragskolben, der in dem Behälter axial beweglich zwischen dem Verschluss und dem Förderkolben angeordnet ist, wobei der Austragskolben mindestens eine flüssigkeitsdurchlässige und für Pulverpartikel undurchlässige Verbindung zwischen den beiden Stirnseiten besitzt,
g) mindestens einen Monomerflüssigkeitsbehälter, der zwischen dem Austragskolben und dem Förderkolben in dem Behälter angeordnet ist,
h) einen Innenraum (den Innenraum der Kartusche), in dem das Zementpulver angeordnet ist, wobei der Innenraum durch die Innenwand des Behälters, den Verschluss und den Austragskolben begrenzt ist, wobei
i) am Austragskolben an der zum Kartuschenkopf zeigenden Stirnseite ein Hohlzylinder angeordnet ist, dessen äußere Mantelfläche an der Kartuscheninnenwand anliegt, wobei der Hohlzylinder eine Höhe von mindestens 3,0 mm, bevorzugt von mindestens 5,0 mm, in axialer Richtung und eine Wandstärke von mindestens 1,0 mm hat.

Der Behälter umfasst dabei die Kartusche als den vorderen Teil des Behälters, in dem das Zementpulver angeordnet ist, und eine Aufnahme als den hinteren Teil des Behälters, in dem der Monomerflüssigkeitsbehälter angeordnet ist.

Ein Verfahren kann beispielsweise mit der beispielhaften Vorrichtung zur Vermischung des Zementpulvers mit der Monomerflüssigkeit unter Bildung von Knochenzementteig mit den folgenden aufeinanderfolgenden Schritten umgesetzt werden:
a) Verbinden der Auspressvorrichtung mit dem Verbindungselement des Behälters,
b) Vortreiben der Stange der Auspressvorrichtung,
c) Verschieben des Förderkolbens in Richtung des Kartuschenkopfs,
d) Komprimierung des mindestens einen Monomerflüssigkeitsbehälters zwischen dem Austragskolben und dem Förderkolben,
e) Bersten oder Reißen des Monomerflüssigkeitsbehälters,
f) Zusammenschieben des geborstenen oder gerissenen Monomerflüssigkeitsbehälters und Auspressen der Luft aus dem Innenraum der Aufnahme und der Monomerflüssigkeit mit dem Förderkolben durch die mindestens eine Verbindung des Austragskolbens in das Zementpulver in den Innenraum der Kartusche,
g) weiteres Zusammenschieben des Monomerflüssigkeitsbehälters und Auspressen der Monomerflüssigkeit mit dem Förderkolben durch die flüssigkeitsdurchlässige Verbindung im Austragskolben und Einleitung der Monomerflüssigkeit durch den Hohlzylinder in das Zementpulver in den Innenraum der Kartusche,
h) Ausbreitung der Monomerflüssigkeit in dem Zementpulver unter gleichzeitiger Verdrängung der Luft aus den Zwischenräumen der Zementpulverpartikel,
i) Benetzung der Zementpulverpartikel mit der Monomerflüssigkeit,
j) Entweichen der Luft aus dem Zementpulver durch den gasdurchlässigen Verschluss,
k) Anquellen der Zementpulverpartikel durch die Monomerflüssigkeit und Auslösung der radikalischen Polymerisation der Monomerflüssigkeit durch Reaktion des Beschleunigers mit dem Initiator,
l) Bildung des Knochenzementteigs aus dem Zementpulver und der Monomerflüssigkeit,
m) Öffnung des Verschlusses in der Austragsöffnung durch axiale Druckbeaufschlagung durch den axial in Richtung Kartuschenkopf gepressten Knochenzementteig,
n) Auspressen des Knochenzementteigs durch die Austragsöffnung infolge der Vorwärtsbewegung des Austragskolbens und des Förderkolbens, und
o) Axiale Bewegung des Austragskolbens in Richtung des Kartuschenkopfs, bis der Hohlzylinder auf den Kartuschenkopf trifft und eine weitere Bewegung des Austragskolbens blockiert.

Wenn der Hohlzylinder die weitere Bewegung des Austragskolbens blockiert, verbleibt in dem vom Hohlzylinder begrenzten Raum im Innenraum der Kartusche ein Rest des Gemischs beziehungsweise des Knochenzementteigs zurück.

Eine beispielhafte Variante des Verfahrens ist durch die folgenden Schritte nach dem Schritt I) des zuvor beschriebenen Verfahrens charakterisiert:
I1) Auspressen des Verschlusses aus der Austragsöffnung, und
I2) Herausfallen des Verschlusses aus der Austragsöffnung oder einem Austragsrohr.

Im Folgenden werden weitere Ausführungsbeispiele der Erfindung anhand von neun schematisch dargestellten Figuren erläutert, ohne jedoch dabei die Erfindung zu beschränken. Dabei zeigt:
Figur 1: eine schematische Querschnittansicht einer beispielhaften erfindungsgemäßen Vorrichtung zum Lagern und Mischen einer Monomerflüssigkeit und eines Zementpulvers;
Figur 2: eine schematische Seitenansicht der Vorrichtung nach Figur 1;
Figur 3: vier schematische Querschnittansichten der Vorrichtung nach den Figuren 1 und 2 mit einer angeschlossenen Auspressvorrichtung übereinander zur Verdeutlichung des Ablaufs eines erfindungsgemäßen Verfahrens;
Figur 4: eine schematische Querschnittansicht als Ausschnittvergrößerung durch den vorderen Teil der erfindungsgemäßen Vorrichtung nach den Figuren 1 bis 3 mit vorgeschobenem Porenfilter;
Figur 5: eine schematische perspektivische Ansicht auf die Teile einer erfindungsgemäßen Vorrichtung mit Applikationsrohr und einer Austragsrohrverlängerung;
Figur 6: eine schematische perspektivische Querschnittansicht der Vorrichtung mit dem Applikationsrohr und der Austragsrohrverlängerung nach Figur 5;
Figur 7: eine schematische Querschnittansicht als Ausschnittvergrößerung der Vorrichtung nach der zweiten Abbildung von oben von Figur 3 beim Einpressen der Monomerflüssigkeit;
Figur 8: eine schematische Querschnittansicht als Ausschnittvergrößerung der Vorrichtung nach der dritten Abbildung von oben von Figur 3 beim nach vorne Pressen des Knochenzementteigs; und
Figur 9: eine schematische Querschnittansicht als Ausschnittvergrößerung der Vorrichtung nach der letzten Abbildung von oben von Figur 3 beim Austragen des Knochenzementteigs.

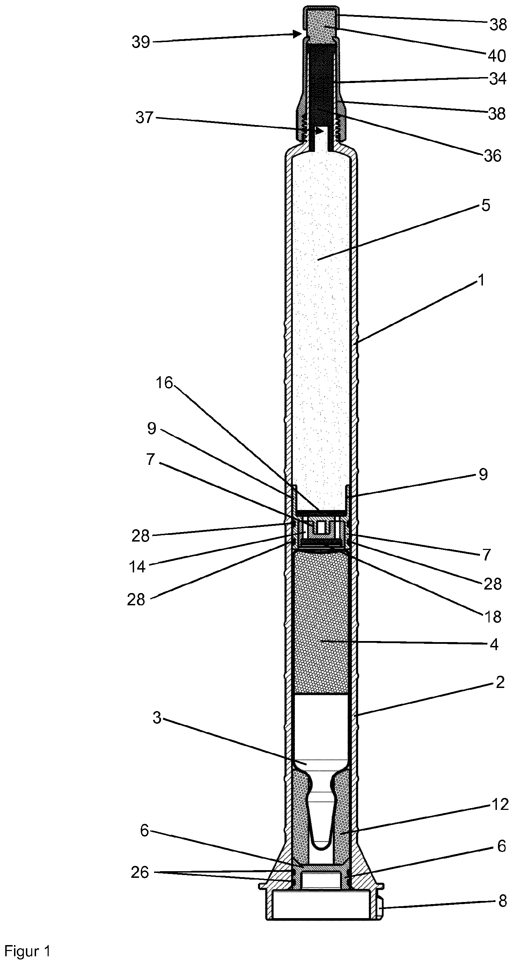

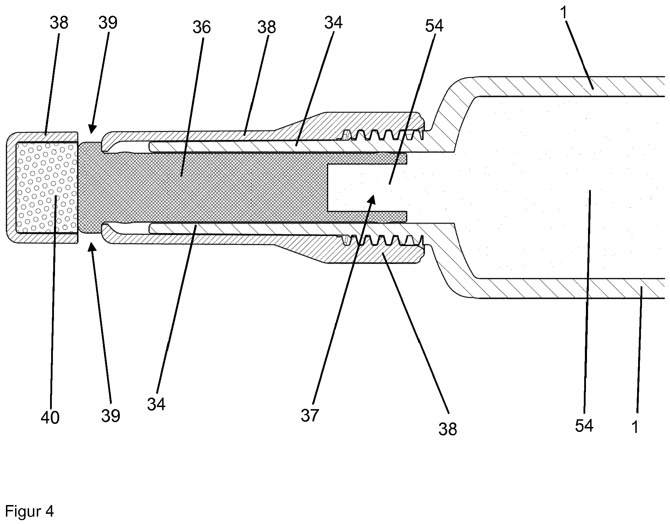

In den Figuren 1 bis 9 sind Abbildungen einer erfindungsgemäßen Vorrichtung gezeigt. Die Figuren 1 bis 3 und 5 und 6 zeigen verschiedene schematische Gesamtansichten der beispielhaften erfindungsgemäßen Vorrichtung. Die Figuren 4 und 7 bis 9 zeigen schematische Querschnittansichten als Detailansichten in Form von Ausschnittvergrößerungen durch verschiedene Bereiche der erfindungsgemäßen Vorrichtung.

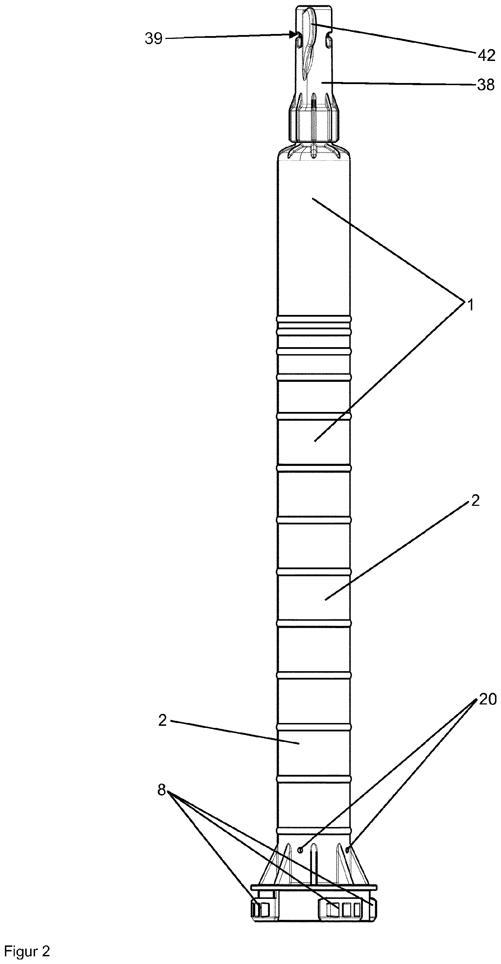

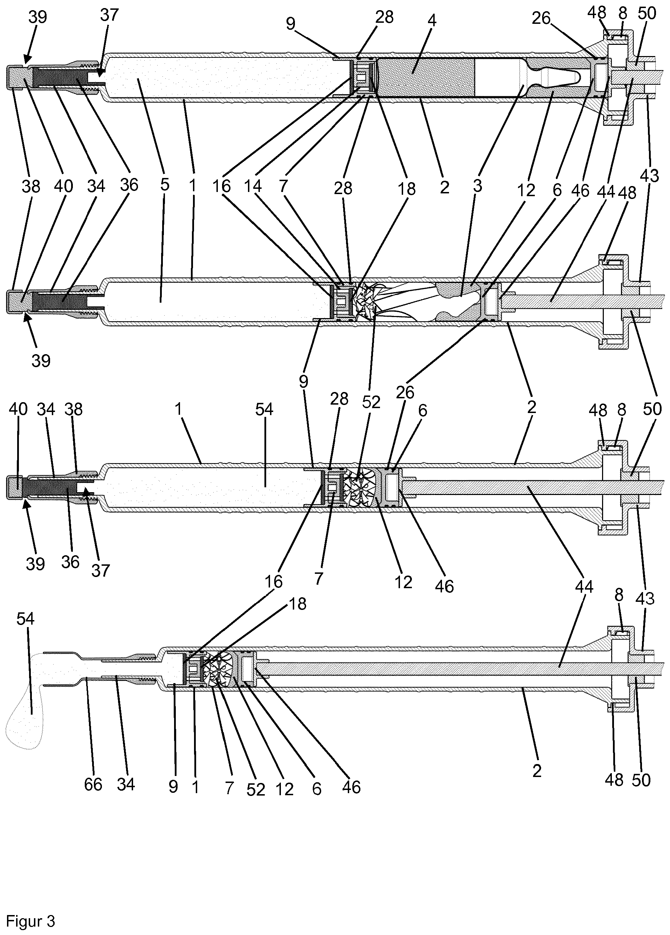

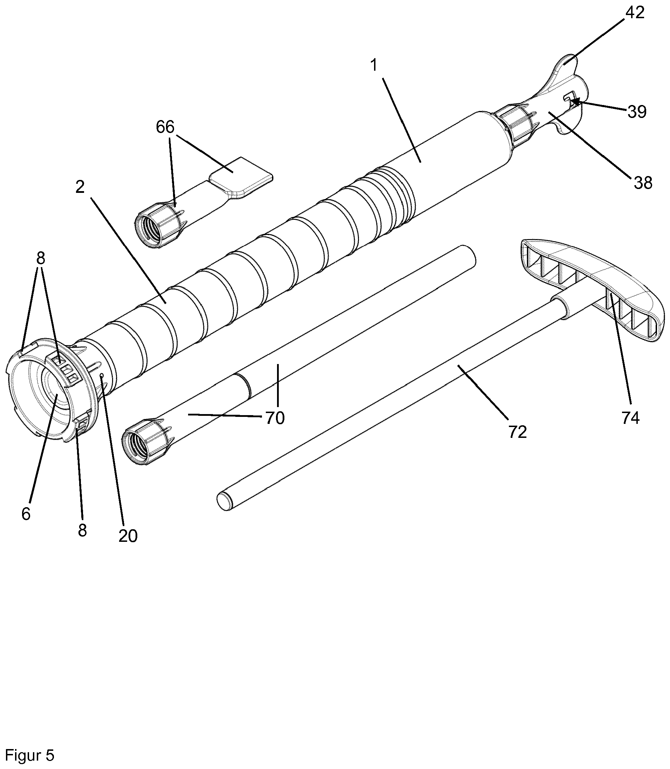

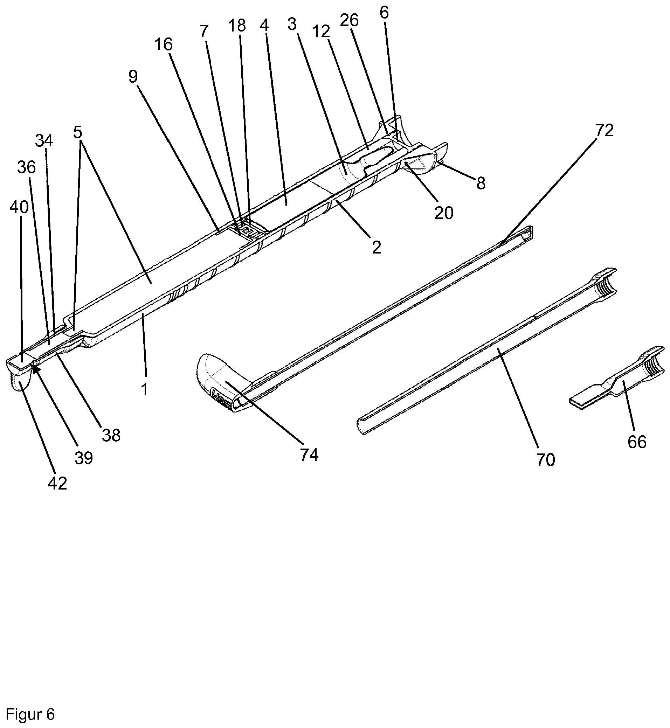

Die erfindungsgemäße Vorrichtung besteht im Wesentlichen aus einem röhrenförmigen Behälter aus Kunststoff, der als vorderen Teil (in den Figuren 1 und 2 oben, in den Figuren 3, 4, 7 bis 9 links, in Figur 5 rechts oben und in Figur 6 links unten) eine Kartusche 1 mit einem zylindrischen Innenraum bildet und der als hinteren Teil eine Aufnahme 2 für eine Glasampulle 3 als Monomerflüssigkeitsbehälter bildet. Anstelle der Glasampulle 3 kann auch ohne weiteres eine aufbrechbare Kunststoffampulle verwendet werden oder es kann durch geringe Umbaumaßnahmen auch ein aufreißbarer Folienbeutel aus einem Metallbeschichteten Kunststoff anstelle der Glasampulle 3 verwendet werden.

Die Rückseite der Vorrichtung ist in den Figuren 1 und 2 unten, in den Abbildungen der Figur 3 rechts, in Figur 5 unten links und in Figur 6 oben rechts gezeigt. Die Röhrenform des Behälters ist besonders gut in den Querschnittansichten der Figuren 1, 3 und 6 zu erkennen. Sowohl der Innenraum der Kartusche 1 als auch der Innenraum der Aufnahme 2 sind zylindrisch mit kreisförmiger Grundfläche. Dabei sind die Durchmesser des Innenraums der Kartusche 1 und der Durchmesser des Innenraums der Aufnahme 2 gleich groß und fluchten. Der Behälter mit der Aufnahme 2 und der Kartusche 1 wird vorzugsweise mit Hilfe einer Spritzgusstechnik aus Kunststoff hergestellt. Die Aufnahme 2 weist also einen zylindrischen Innenraum auf, in den die Glasampulle 3 eingesteckt ist. In der Glasampulle 3 befindet sich die Monomerflüssigkeit 4. In Figur 1 ist die Vorrichtung umgedreht gezeigt, so dass die Gravitation nach oben wirkt und sich die Monomerflüssigkeit 4 im oberen Teil der Glasampulle 3 sammelt. In den Innenraum der Kartusche 1 ist ein Zementpulver 5 eingefüllt oder vorzugsweise eingepresst. Die Monomerflüssigkeit 4 und das Zementpulver 5 bilden die Ausgangskomponenten für einen PMMA-Knochenzement, der mit der Vorrichtung hergestellt werden kann. Aufgrund der Glasampulle 3 kann die Monomerflüssigkeit 4 sehr lange in der Aufnahme 2 und dadurch in der Vorrichtung gelagert werden. Das Zementpulver 5 kann ebenfalls über längere Zeiträume in der Vorrichtung gelagert werden. Die Vorrichtung ist damit zum Lagern der Monomerflüssigkeit 4 und des Zementpulvers 5 als Ausgangskomponenten eines Knochenzementteigs des PMMA-Knochenzements geeignet. Die Vorrichtung ist aber auch zum Mischen des Knochenzementteigs aus den Ausgangskomponenten und zum Austragen des gemischten Knochenzementteigs geeignet und vorgesehen.

In der Aufnahme 2 ist ein im zylindrischen Innenraum der Aufnahme 2 in Längsrichtung beweglicher Förderkolben 6 aus Kunststoff angeordnet. Der Förderkolben 6 ist im Bereich der Rückseite der Aufnahme 2 angeordnet. Die Glasampulle 3 kann mit dem Förderkolben 6 in der Aufnahme 2 zusammengedrückt und dabei zersplittert werden, indem der Förderkolben 6 in Richtung der Vorderseite, also in Richtung der Kartusche 1 gedrückt wird. Der Förderkolben 6 weist an der Vorderseite Abstreifer auf, mit denen Splitter der Glasampulle 3 von der Innenwand der Aufnahme 2 abgestreift werden. Die Abstreifer liegen hierzu seitlich an der Innenwand des Innenraums der Aufnahme 2 an.

In dem Innenraum der Kartusche 1 ist in dessen Rückseite (in den Figuren 1 und 2 Richtung unten, in den Figuren 3, 4 und 7 bis 9 Richtung rechts) ein Austragskolben 7 aus Kunststoff angeordnet. An der Rückseite der Aufnahme 2 ist ein Befestigungsmittel 8 vorgesehen, mit dem die Aufnahme 2 an einer Auspressvorrichtung 43 (in Figur 1 und 2 nicht zu sehen, siehe aber Figur 3) verbunden werden kann. Das Befestigungsmittel 8 ist vorzugsweise zur Bildung eines Bajonettverschlusses 8 geeignet und vorgesehen. Dadurch kann der Förderkolben 6, der von der Rückseite der Aufnahme 2 aus frei zugänglich ist, mit der Auspressvorrichtung 43 in Richtung der Vorderseite der Kartusche 1 vorgetrieben werden.

Der Austragskolben 7 weist an seiner Vorderseite einen Hohlzylinder 9 zum Verlängern der Strecke auf, die die Monomerflüssigkeit 4 durch das Zementpulver 5 fließen muss, bis sie zur Innenwand der Kartusche 1 gelangt. Zudem dient der Hohlzylinder 9 zur Beabstandung des Austragskolbens 7 von einer Austragsöffnung an der Vorderseite des Innenraums der Kartusche 1 sowie zum Schaffen eins Totvolumens zwischen dem Austragskolben 7 und der Vorderseite des Innenraums der Kartusche 1, wenn der Austragskolben 7 beziehungsweise der Hohlzylinder 9 maximal an die Vorderseite des Innenraums der Kartusche 1 gedrückt ist. Der Hohlzylinder 9 ist vorliegend rotationssymmetrisch und ist nach Art eines Rohrstücks geformt. Der Hohlzylinder 9 kann aber auch parallel zur Zylinderachse des Hohlzylinders 9 verlaufende Längsschnitte aufweisen. An der Vorderseite ist der Hohlzylinder 9 eben. Der Hohlzylinder 9 ist an seiner zur Austragsöffnung weisenden Vorderseite offen.

In dem Innenraum der Aufnahme 2 ist eine Lagerung 12 aus Schaumstoff vorgesehen, die als Transportsicherung und als Stoßsicherung für die Glasampulle 3 dient. Damit soll verhindert werden, dass die Glasampulle 3 bei Erschütterungen oder Stößen ungewollt aufbricht. Der Schaumstoff und damit die Lagerung 12 sind durchlässig für Gase.

Die Kartusche 1 und die Aufnahme 2 sind einteilig als gemeinsames Kunststoffteil ausgeführt. Die Aufnahme 2 und die Kartusche 1 sind für die Monomerflüssigkeit 4 flüssigkeitsdurchlässig miteinander über eine Verbindung 14 im Austragskolben 7 verbunden. Die Verbindung 14 durch den Austragskolben 7 mündet durch einen für das Zementpulver 5 undurchlässigen aber für die Monomerflüssigkeit 4 durchlässigen Porenfilter 16 in den Innenraum der Kartusche 1.

In der Einmündung zur Verbindung 14 ist in dem Austragskolben 7 ein Filter 18 angeordnet, mit dem die Splitter der Glasampulle 3 zurückgehalten werden können. Statt dem Filter 18 oder zusätzlich zum Filter 18 kann auch ein Sieb vorgesehen sein.

Mehrere Belüftungsöffnungen 20 sind in der Wandung der Aufnahme 2 vorgesehen, durch die der Innenraum der Aufnahme 2 mit Hilfe eines sterilisierenden Gases wie Ethylenoxid sterilisiert werden kann. Die Lagerung 12 ist ebenfalls gasdurchlässig und verschließt daher nicht die Belüftungsöffnungen 20. Die Belüftungsöffnungen 20 sind unmittelbar benachbart zum Förderkolben 6 angeordnet, so dass der Förderkolben 6 sich unmittelbar vor die Belüftungsöffnungen 20 schiebt und somit die Belüftungsöffnungen 20 unmittelbar verschließt, wenn der Förderkolben 6 in Richtung der Kartusche 1 vorgetrieben wird. Dadurch wird verhindert, dass Monomerflüssigkeit 4 durch die Belüftungsöffnungen 20 austreten kann, wenn die Glasampulle 3 in der Aufnahme 2 geöffnet wurde.

Der zylindrische Förderkolben 6 hat einen zur Zylindergeometrie des Innenraums der Aufnahme 2 passenden Außenumfang und ist über zwei umlaufende Dichtungen 26 gegen die Innenwand der Aufnahme 2 flüssigkeitsdicht abgedichtet. Ebenso ist der Austragskolben 7 über zwei umlaufende Dichtungen 28 gegen die Innenwand der Kartusche 1 flüssigkeitsdicht abgedichtet. Diese Dichtungen 26, 28 dienen dazu, einen Austritt von Monomerflüssigkeit 4 oder von Knochenzement zu verhindern, um eine Kontamination der Umgebung (des OP-Saals und des Anwenders) zu verhindern. Die Dichtungen 26, 28 können hierzu aus Gummi bestehen.

Der Innenraum der Kartusche 1 mündet an der Vorderseite in ein Austragsrohr 34, das die Austragsöffnung der Kartusche 1 begrenzt. Das Austragsrohr 34 weist an seiner Basis ein Außengewinde auf. Im Inneren der Austragsrohrs 34 ist ein Porenfilter 36 als Verschluss für die Kartusche 1 angeordnet. Der Porenfilter 34 ist für das Zementpulver 5 undurchlässig aber für Gase durchlässig. In der Rückseite des Porenfilters 36 ist eine Vertiefung 37 vorgesehen. Das Zementpulver 5 ist auch in der Vertiefung 37 enthalten. An dem Außengewinde des Austragsrohrs 34 ist eine Kappe 38 befestigt, wobei der vordere Teil der Kappe 38 mit einem Styropor oder Schaumstoff 40 gefüllt ist. An der Kappe 38 sind zwei Flügel 42 vorgesehen, so dass die Kappe 38 nach Art einer Flügelschraube bequem mit der Hand von dem Austragsrohr 34 abgeschraubt werden kann. Die Kappe 38 weist seitliche Öffnungen 39 auf. Durch diesen Aufbau können das Innere der Kartusche 1 und das Zementpulver 5 mit Hilfe von Ethylenoxid sterilisiert werden, da die Öffnungen 39 in der Kappe 38, das Styropor oder der Schaumstoff 40, der Porenfilter 36 und die Zwischenräume zwischen den Pulverpartikeln des Zementpulvers 5 luftdurchlässig sind. Gleichzeitig kann Luft aus der Aufnahme 2 durch das Zementpulver 5, den Porenfilter 36, das Styropor oder der Schaumstoff 40 und die Öffnungen 39 in der Kappe 38 herausgepresst werden, wenn der Förderkolben 6 in Richtung der Aufnahme 1 gepresst wird. Die Kappe 38 bildet zusammen mit dem Styropor oder Schaumstoff 40 und mit dem Porenfilter 36 einen Verschluss für die Austragsöffnung der Kartusche 1 beziehungsweise für das Austragsrohr 34.

Das Zementpulver 5 ist in der Kartusche 1 eingeschlossen, da alle Öffnungen 39 und Verbindungen 14 mit Hilfe der Porenfilter 16, 36 für das Zementpulver 5 undurchlässig verschlossen sind. Der Inhalt der Kartusche 1 kann dabei durch Evakuieren und Spülen mit Ethylenoxid sterilisiert werden. Dadurch ist die Vorrichtung auch zum langfristigen Lagern des Zementpulvers 5 geeignet.

In den Figuren 5 und 6 sind neben der Vorrichtung auch das Applikationsrohr 66 und die Austragsrohrverlängerung 70 für die Vorrichtung gezeigt, die jeweils alternativ zueinander anstelle der Kappe 38 auf das Austragsrohr 34 aufgeschraubt werden können. Dazu weisen das Applikationsrohr 66 und die Austragsrohrverlängerung 70 ein zu dem Außengewinde des Austragsrohrs 34 passendes Innengewinde auf. Die Austragsrohrverlängerung 70 kann mit einem Verschluss 72 verschlossen sein. Der Verschluss 72 endet in einem Griff 74, mit dem die Austragsrohrverlängerung 70 bequem mit der Hand auf das Austragsrohr 34 geschraubt werden kann, wenn der Verschluss 72 in der Austragsrohrverlängerung 70 steckt. Zudem kann mit dem Griff 74 der Verschluss 72, der die der Kartusche 1 zugewandte Seite der Austragsrohrverlängerung 70 verschließt, bequem entfernt werden, auch wenn die Austragsrohrverlängerung 70 mit dem Austragsrohr 34 fest verschraubt ist.

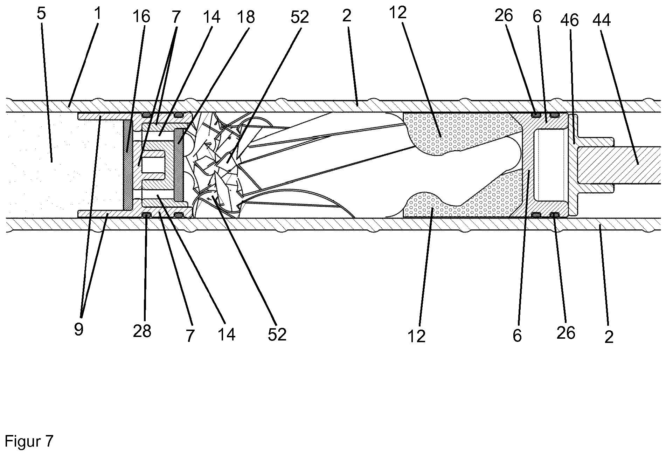

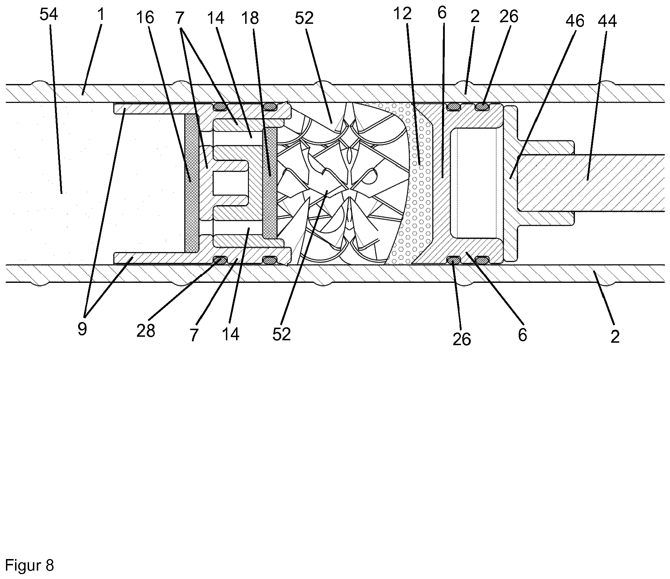

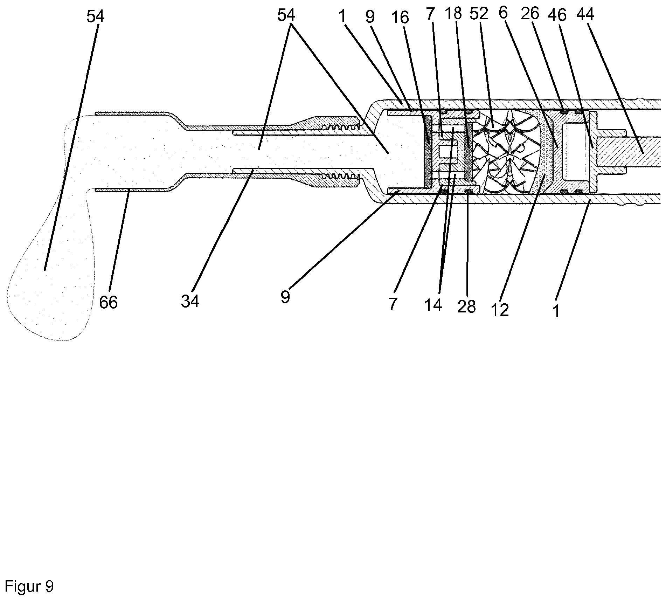

Figur 3 zeigt vier schematische Querschnittansichten der erfindungsgemäßen Vorrichtung nach den Figuren 1 und 2 übereinander zur Verdeutlichung des Ablaufs eines erfindungsgemäßen Verfahrens. Dazu zeigt Figur 4 eine Ausschnittvergrößerung der dritten Abbildung der Figur 3, Figur 7 eine Ausschnittvergrößerung der zweiten Abbildung von oben der Figur 3, Figur 8 eine Ausschnittvergrößerung der vierten Abbildung von oben der Figur 3 und Figur 9 eine Ausschnittvergrößerung der untersten Abbildung der Figur 3.

Zu Beginn des Verfahrens liegt die Vorrichtung im Ausgangszustand vor, so wie sie auch in Figur 1 gezeigt ist. In diesem Zustand wird die Vorrichtung in eine Auspressvorrichtung 43 eingesetzt, beispielsweise eine herkömmliche mit der Hand antreibbare Kartuschenpistole. Diese Situation ist in der obersten Abbildung von Figur 3 gezeigt. Die Auspressvorrichtung 43 umfasst eine linear vortreibbare Stange 44. Von der Auspressvorrichtung 43 ist nur der vordere Teil dargestellt. Die Auspressvorrichtung 43 umfasst auch einen Griff und einen Kipphebel (in den Abbildungen nicht zu sehen) zum manuellen Antreiben der Stange 44 der Auspressvorrichtung 43, wie bei herkömmlichen manuell angetriebenen Auspressvorrichtungen auch. Die Vorrichtung wird mit dem Befestigungsmittel 8 an der Auspressvorrichtung 43 befestigt (siehe oberste Abbildung in Figur 3). An der Spitze der Stange 44 ist ein flacher Teller 46 zum Antreiben des Förderkolbens 6 vorgesehen. Die Stange 44 drückt mit dem Teller 46 auf den Förderkolben 6, wenn die Stange 44 von der Auspressvorrichtung 43 in die Aufnahme 2 hineingedrückt wird. Die Auspressvorrichtung 43 ist hierzu über ein Gegenbefestigungsmittel 48 an die Rückseite der Aufnahme 2 angeschlossen, so dass der Teller 46 beim Vortreiben der Stange 44 auf den Förderkolben 6 drückt und diesen in Richtung der Kartusche 1 vortreibt. Hierzu ist die Stange 44 gegen ein Lager 50 und darüber gegen das Gegenbefestigungsmittel 48 und damit gegen die Aufnahme 2 linear beweglich gelagert.

Die Auspressvorrichtung 43 wird bedient und dabei die Stange 44 und mit der Stange 44 der Förderkolben 6 in Richtung der Kartusche 1 vorgetrieben. Zu Beginn der Bewegung des Förderkolbens 6 verschließt dieser die Belüftungsöffnungen 20. Die Lagerung 12 wird komprimiert und der Förderkolben 6 trifft auf den Kopf der Glasampulle 3. Da die Glasampulle 3 an der Vorderseite an dem Austragskolben 7 anliegt und sich der Innenraum der Aufnahme 2 weiter verkleinert, wird die Glasampulle 3 zerbrochen. Die Monomerflüssigkeit 4 tritt aus der Glasampulle 3 in den Innenraum der Aufnahme 2 aus. Der Austragskolben 7 kann nicht oder nicht weit von der Glasampulle 3 in Richtung des Porenfilters 36 geschoben werden, wenn das Zementpulver 5 trocken ist, also nicht von der Monomerflüssigkeit 4 benetzt ist, da das trockene Zementpulver 5 nicht fließfähig ist und eine Bewegung des Austragskolbens 7 blockiert. Diese Situation ist in Figur 3, zweite Abbildung von oben, sowie in der vergrößerten Ausschnittansicht in Figur 7 gezeigt. Überstehende Luft aus der Aufnahme 2 wird durch den Filter 18, die Verbindung 14, den Porenfilter 16, durch die Zwischenräume zwischen den Partikeln des Zementpulvers 5, durch den Porenfilter 36, durch den Schaumstoff 40 und aus den Öffnungen 39 in der Kappe 38 aus der Vorrichtung herausgedrückt.

Von der Glasampulle 3 bleiben schließlich nur kleine Splitter 52 zurück, die von dem Filter 18 zurückgehalten werden und in dem röhrenförmigen Behälter verbleiben. Die Monomerflüssigkeit 4 wird durch den Filter 18, die Verbindung 14 und den Porenfilter 16 in das Zementpulver 5 gepresst und beginnt dort mit dem Zementpulver 5 zu reagieren, so dass sich aus dem Gemisch 54 der Knochenzementteig 54 bildet. Dabei kann die Monomerflüssigkeit 4 nicht direkt von dem Porenfilter 16 aus zur Innenwand der Kartusche 1 fließen, da diese vollständig oder im Fall eines geschlitzten Hohlzylinders 9 weitgehend von dem Hohlzylinder 9 abgedeckt ist. Dadurch wird die Monomerflüssigkeit 4 gezwungen sich einen Weg durch das Zementpulver 5 zu bahnen. Monomerflüssigkeitsblasen oder Monomerflüssigkeitsansammlungen können so verhindert werden und es wird ein homogenerer Knochenzementteig 54 gemischt als ohne Verwendung des Hohlzylinders 9.

Die Menge der Monomerflüssigkeit 4 ist so gewählt, dass das Zementpulver 5 bis in die vorderste Spitze der Kartusche 1, das heißt bis in die Vertiefung 37 im Porenfilter 36 mit der Monomerflüssigkeit 4 benetzt wird. Diese Situation ist in Figur 3, dritte Abbildung von oben und in der Detailansicht nach Figur 8 gezeigt. Der Porenfilter 36 wird, sobald das Gemisch 54 entstanden ist, von dem auf das Gemisch 54 aufgrund des Drucks auf den Austragskolben 7 wirkenden Druck nach vorne getrieben und komprimiert den Schaumstoff 40. Wenn der Porenfilter 36 nun nach vorne rutscht, so wird er von außen für den Anwender durch die Öffnung 39 in der Kappe 38 sichtbar. Diese Situation ist in Figur 4 im Detail gezeigt. Hierzu hat der Porenfilter 36 vorzugsweise eine andere Farbe und/oder Helligkeit als der Schaumstoff 40. Beispielsweise kann der Schaumstoff 40 weiß sein und der Porenfilter 36 rot.

In diesem Zustand wird die Kappe 38 mit dem Porenfilter 36 und dem Schaumstoff 40 abgeschraubt und stattdessen eine verlängerte Austragsöffnung in Form eines Applikatorrohrs 66 oder in Form einer Austragsrohrverlängerung 70 auf das Austragsrohr 34 aufgeschraubt (siehe auch Figur 5 und Figur 6). Beim Abschrauben der Kappe 38 wird der vorderste Teil des Gemischs 54 beziehungsweise des Knochenzementteigs 54, der sich in der Vertiefung 37 des Porenfilters 38 befindet, mit der Kappe 38 und dem Porenfilter 36 entfernt. Dadurch wird ein potenziell schlechter gemischter Teil des Knochenzementteigs 54 entfernt und somit eine größere Homogenität des verfügbaren Knochenzementteigs 54 erreicht.

Durch weiteres Vortreiben der Stange 44 wird der Förderkolben 6, die Scherben 52 und der davor angeordnete Austragskolben 7 angetrieben. Über das Applikatorrohr 66 wird dann der Knochenzementteig 54 aus der Kartusche 1 ausgetragen. Dazu wird der Austragskolben 7 mit der Stange 44 in Richtung des Austragsrohrs 34 vorgetrieben (siehe hierzu auch die vierte Abbildung von oben der Figur 3 sowie die Detailansicht nach Figur 9). Der Knochenzementteig 54 aus dem Inneren der Kartusche 1 wird durch das Austragsrohr 34 und das Applikatorrohr 66 ausgetrieben und kann dort appliziert werden oder theoretisch zur weiteren Verarbeitung verwendet werden.

Schließlich trifft der Hohlzylinder 9 auf den Kartuschenkopf beziehungsweise die Vorderseite des Innenraums der Kartusche 1. Da am Ende des Auspressvorgangs der Austragskolben 7 blockiert wird, kann es dazu kommen, dass die Scherben und Splitter 52 der Glasampulle 3 durch den auf die Scherben und Splitter 52 wirkenden wachsenden Druck noch weiter komprimiert werden und dabei noch weitere Reste der Monomerflüssigkeit 4 aus dem Zwischenraum zwischen dem Austragskolben 7 und dem Förderkolben 6 in den vorderen Teil der Kartusche 1 gedrückt werden. Dadurch kann es zu einer Veränderung der Zusammensetzung des Knochenzementteigs 54 kommen, da der Anteil an flüssiger Monomerflüssigkeit 4 in der Mischung 54 erhöht wird. Wenn der Knochenzementteig 54 schon sehr weitgehend reagiert hat, kann es auch sein, dass sich die Monomerflüssigkeit 4 einen Weg an dem Knochenzementteig 54 vorbei bahnt. Der Hohlzylinder 9 weist eine Höhe von 3 mm, bevorzugt von 5 mm oder größer auf, so dass durch den dadurch erzeugten Abstand gewährleistet ist, dass die Vorderseite des Austragskolbens 7 von der Vorderseite des Innenraums der Kartusche 1 beabstandet ist, wenn der Austragskolben 7 so weit nach vorne gedrückt es, wie es mit einer manuell angetriebenen Auspressvorrichtung 43 möglich ist. Dadurch entsteht im Innenraum der Kartusche 1 und zwar in dem von dem Hohlzylinder 9 begrenzten Bereich ein Totvolumen, das nicht aus der Kartusche 1 durch die Austragsöffnung und das Austragsrohr 34 ausgetrieben werden kann.

In diesem Totvolumen befindet sich nun der Teil des Knochenzementteigs 54, der gegebenenfalls einen zu großen Anteil an Monomerflüssigkeit 4 enthält. Auch wenn nachfolgend immer weiter gepresst wird, kann kein weiterer Knochenzementteig 54 aus dem Totvolumen aus der Vorrichtung ausgepresst werden. Durch diesen Aufbau wird sichergestellt, dass kein Knochenzementteig 54 mit einer sich ändernden Konsistenz aufgrund sich ändernder Zusammensetzung mit der Vorrichtung appliziert werden kann.

Die Öffnungen 39 dienen auch als visuelle Marker anhand derer festgestellt werden kann, wann die Vorrichtung einsatzbereit ist. Wenn der Porenfilter 36 aufgrund des Drucks des Knochenzementteigs 54 nach vorne gedrückt wird und dabei das Styropor 40 in der Kappe 38 zusammenpresst, wird der Porenfilter 36 durch die Öffnungen 39 sichtbar. Dadurch kann der Anwender erkennen, dass der Knochenzementteig 54 fertig gemischt in der Kartusche 1 vorliegt und damit einsatzbereit ist. Der Anwender kann zu diesem Zeitpunkt die Kappe 38 mit dem Porenfilter 36 abschrauben und das Applikatorrohr 66 oder die Austragsrohrverlängerung 70 auf das Austragsrohr 34 aufschrauben. Anschließend kann der Austragskolben 7 über den Förderkolben 6 mit der Stange 44 angetrieben werden und dadurch der Knochenzementteig 54 aus der Kartusche 1 durch das Applikatorrohr 66 oder die Austragsrohrverlängerung 70 ausgetrieben werden.

### Bezugszeichenliste

- 1: Kartusche
- 2: Aufnahme
- 3: Ampulle
- 4: Monomerflüssigkeit
- 5: Zementpulver
- 6: Förderkolben
- 7: Austragskolben
- 8: Befestigungsmittel / Bajonettverschluss
- 9: Hohlzylinder
- 12: Lagerung
- 14: Verbindung
- 16: Porenfilter
- 18: Filter
- 20: Belüftungsöffnung
- 26: Dichtung
- 28: Dichtung
- 34: Austragsrohr
- 36: Porenfilter
- 37: Vertiefung
- 38: Kappe
- 39: Öffnung
- 40: Schaumstoff
- 42: Flügel
- 43: Auspressvorrichtung / Kartuschenpistole
- 44: Stange
- 46: Teller
- 48: Gegenbefestigungsmittel / Bajonettverschluss
- 50: Lagerung
- 52: Splitter
- 54: Knochenzementteig / Gemisch
- 66: Applikatorrohr
- 70: Austragsrohrverlängerung
- 72: Verschluss
- 74: Griff

## Patentansprüche

1. Vorrichtung zum Herstellen eines Knochenzementteigs (54) aus einer Monomerflüssigkeit (4) und einem Zementpulver (5) als Ausgangskomponenten des Knochenzementteigs (54) und zum Austragen des gemischten Knochenzementteigs (54), die Vorrichtung aufweisend
eine Kartusche (1) mit einem zylindrischen Innenraum, wobei der Innenraum der Kartusche (1) an einer Vorderseite bis auf eine Austragsöffnung zum Austreiben des Knochenzementteigs (54) verschlossen ist, wobei im Innenraum der Kartusche (1) ein in Richtung der Austragsöffnung drückbarer Austragskolben (7) angeordnet ist und wobei das Zementpulver (5) im Innenraum der Kartusche (1) zwischen der Austragsöffnung und dem Austragskolben (7) angeordnet ist, wobei an einer der Austragsöffnung zugewandten Vorderseite des Austragskolbens (7) ein Hohlzylinder (9) angeordnet ist, wobei der Hohlzylinder (9) an seiner der Austragsöffnung zugewandten Vorderseite offen ist und sich von der Vorderseite des Austragskolbens (7) zumindest 3 mm in den Innenraum der Kartusche (1) erstreckt, wobei
in dem Austragskolben (7) zumindest eine Verbindung (14) von der Rückseite des Austragskolbens (7) zur Vorderseite des Austragskolbens (7) zum Einleiten der Monomerflüssigkeit (4) in den Innenraum der Kartusche (1) vorgesehen ist, wobei die zumindest eine Verbindung (14) für die Monomerflüssigkeit (4) und Gase durchlässig ist und für das Zementpulver (5) undurchlässig ist und wobei die zumindest eine Verbindung (14) aus dem Austragskolben (7) innerhalb des Hohlzylinders (9) oder durch Leitungen in dem Hohlzylinder (9) an der Vorderseite des Hohlzylinders (9) in den Innenraum der Kartusche (1) münden.

2. Vorrichtung nach Anspruch 1, **dadurch gekennzeichnet, dass**
der Hohlzylinder (9) an seiner äußeren Mantelfläche maximal 0,5 mm von der Innenwand des Innenraums der Kartusche (1) beabstandet ist, bevorzugt maximal 0,1 mm von der Innenwand des Innenraums der Kartusche (1) beabstandet ist.

3. Vorrichtung nach Anspruch 1, **dadurch gekennzeichnet, dass**
der Hohlzylinder (9) zumindest bereichsweise an der Innenwand des Innenraums der Kartusche (1) anliegt, vorzugsweise an seiner äußeren Mantelfläche an der Innenwand des Innenraums der Kartusche (1) anliegt.

4. Vorrichtung nach einem der vorangehenden Ansprüche, **dadurch gekennzeichnet, dass**
der Hohlzylinder (9) eine weitere Bewegung des Austragskolbens (7) in Richtung der Vorderseite der Kartusche (1) blockiert, wenn die Vorderseite des Hohlzylinders (9) an der Vorderseite des Innenraums der Kartusche (1) anliegt, so dass der Austragskolben (7) von der Vorderseite des Innenraums der Kartusche (1) beabstandet ist und ein Totvolumen in dem Innenraum der Kartusche (1) verbleibt.

5. Vorrichtung nach einem der vorangehenden Ansprüche, **dadurch gekennzeichnet, dass**
der Hohlzylinder (9) zumindest einen Schlitz aufweist, vorzugsweise zumindest einen parallel zur Zylinderachse des Hohlzylinders (9) verlaufenden Schlitz aufweist, besonders bevorzugt zumindest einen von der Vorderseite bis zum Austragskolben (7) reichenden Schlitz aufweist.

6. Vorrichtung nach einem der vorangehenden Ansprüche, **dadurch gekennzeichnet, dass**
in dem von dem Hohlzylinder (9) umschlossenen Teil des Innenraums der Kartusche (1) Zementpulver (5) enthalten ist, insbesondere eingepresst ist.

7. Vorrichtung nach einem der vorangehenden Ansprüche, **dadurch gekennzeichnet, dass**
das von dem Hohlzylinder (9) begrenzte Volumen des Innenraums der Kartusche (1) zumindest 1 cm³ groß ist, bevorzugt zumindest 3 cm³ groß ist.

8. Vorrichtung nach einem der vorangehenden Ansprüche, **dadurch gekennzeichnet, dass**
der Hohlzylinder (9) sich von der Vorderseite des Austragskolbens (7) zumindest 5 mm in den Innenraum der Kartusche (1) erstreckt, bevorzugt zumindest 7,5 mm in den Innenraum der Kartusche (1) erstreckt, besonders bevorzugt zumindest 10 mm in den Innenraum der Kartusche (1) erstreckt, und/oder
die Wandstärke des Hohlzylinders (9) zumindest 1 mm beträgt, bevorzugt zumindest 1,5 mm beträgt, besonders bevorzugt zumindest 2 mm beträgt.

9. Vorrichtung nach einem der vorangehenden Ansprüche, **dadurch gekennzeichnet, dass**
die Vorrichtung eine Aufnahme (2) aufweist, in der die Monomerflüssigkeit (4), insbesondere ein Monomerflüssigkeitsbehälter (3) enthaltend die Monomerflüssigkeit (4), enthalten ist, wobei die Rückseite der Kartusche (1) mit der Vorderseite der Aufnahme (2) verbunden ist, vorzugsweise derart verbunden ist, dass der Innenraum der Kartusche (1) mit dem Innenraum der Aufnahme (2) fluchtet.

10. Vorrichtung nach Anspruch 9, **dadurch gekennzeichnet, dass** ein Innenraum der Aufnahme (2) und der Innenraum der Kartusche (1) über eine für die Monomerflüssigkeit (4) und für Gase durchlässige aber für das Zementpulver (5) undurchlässige Verbindung (14) miteinander verbunden sind,
die Aufnahme (2) einen zylindrischen Innenraum aufweist, in dem die Monomerflüssigkeit (4), insbesondere ein Monomerflüssigkeitsbehälter (3) enthaltend die Monomerflüssigkeit (4), angeordnet ist,
in der Aufnahme (2) ein in Längsrichtung der Aufnahme (2) beweglicher Förderkolben (6) angeordnet ist, der von einer Rückseite der Aufnahme (2) aus in Richtung der Vorderseite vortreibbar ist, wobei die Monomerflüssigkeit (4), insbesondere ein Monomerflüssigkeitsbehälter (3) enthaltend die Monomerflüssigkeit (4), zwischen dem Förderkolben (6) und dem Austragskolben (7) angeordnet ist, und/oder in der Wandung der Aufnahme (2) zumindest eine Belüftungsöffnung (20) angeordnet ist, die den Innenraum der Aufnahme (2) mit der Umgebung verbindet, wobei die zumindest eine Belüftungsöffnung (20) derart dicht im Bereich des Förderkolbens (6) angeordnet ist, dass sie durch eine Bewegung des Förderkolbens (6) in Richtung der Vorderseite der Aufnahme (2) verschlossen wird, bevor ein in der Aufnahme (2) angeordneter Monomerflüssigkeitsbehälter (3), in dem die Monomerflüssigkeit (4) enthalten ist, durch die Bewegung des Förderkolbens (6) geöffnet ist.

11. Vorrichtung nach einem der vorangehenden Ansprüche, **dadurch gekennzeichnet, dass**
an der Rückseite der Vorrichtung, ein Befestigungsmittel (8) zur Befestigung einer Auspressvorrichtung (43) angeordnet ist, mit der der Austragskolben (7) in Richtung der Austragsöffnung drückbar ist.

12. Vorrichtung nach einem der vorangehenden Ansprüche, **dadurch gekennzeichnet, dass**
die Austragsöffnung an deren Vorderseite mit einem Verschluss (36), insbesondere mit einem Stopfen (36), verschlossen ist, wobei der Knochenzementteig (54) durch die Austragsöffnung aus der Kartusche (1) auspressbar ist, wenn die Austragsöffnung geöffnet ist, und wobei vorzugsweise der Verschluss (36) für Gase durchlässig und für das Zementpulver (5) undurchlässig ist und wobei vorzugsweise der Verschluss (36) an der dem Innenraum der Kartusche (1) zugewandten Rückseite eine Vertiefung (37) aufweist, in der der vorderste Teil des Zementpulvers (5) enthalten ist.

13. Vorrichtung nach einem der vorangehenden Ansprüche, **dadurch gekennzeichnet, dass**
der Querschnitt des Innenraums der Kartusche (1) maximal 16 cm² beträgt, bevorzugt maximal 5 cm² beträgt, und/oder
der Hohlzylinder (9) einteilig mit dem Austragskolben (7) oder mit einem an der Innenwand der Kartusche (1) anliegenden Teil des Austragskolbens (7) ausgebildet ist.

14. Verfahren zur Herstellung eines Knochenzementteigs (54), insbesondere eines pastenförmigen Polymethylmethacrylat-Knochenzementteigs (54), wobei der Knochenzementteig (54) aus einem Zementpulver (5) und einer Monomerflüssigkeit (4) mit einer Vorrichtung nach einem der vorangehenden Ansprüche hergestellt wird, mit den folgenden nacheinander ablaufenden Schritten:
a) Einsetzen der Vorrichtung in eine Auspressvorrichtung (43), die Auspressvorrichtung (43) aufweisend eine axial vortreibbare Stange (44),
a1) Vortreiben eines Förderkolben (6) mit der Stange (44) in Richtung der Kartusche (1) der innerhalb einer an der Rückseite der Kartusche (1) angeordneten Aufnahme (2) an der Rückseite der Aufnahme (2) beweglich gelagert ist, wobei durch die Bewegung des Förderkolbens (6) ein Monomerflüssigkeitsbehälter (3), in dem die Monomerflüssigkeit (4) enthalten ist, geöffnet wird und Eindrücken der Monomerflüssigkeit (4) in den Innenraum der Kartusche (1) durch die Bewegung des Förderkolbens (6), so dass sich die Monomerflüssigkeit (4) mit dem Zementpulver (5) im Innenraum der Kartusche (1) mischt, wobei die Monomerflüssigkeit (4) den Hohlzylinder (9) umfließt, bevor sie bis zur Innenwand der Kartusche (1) gelangt,
b) der Austragskolben (7) wird mit der Stange (44) in Richtung der Austragsöffnung der Kartusche (1) vorgetrieben, wobei durch die Bewegung des Austragskolbens (7) die Mischung aus dem Zementpulver (5) und der Monomerflüssigkeit (4) aus der Kartusche (1) als Knochenzementteig (54) aus der Vorrichtung ausgetrieben wird, und
c) die Vorderseite des Hohlzylinders (9) auf die Vorderseite des Innenraums der Kartusche (1) trifft, wobei mit dem Hohlzylinder (9) eine weitere Bewegung des Austragskolbens (7) in Richtung der Austragsöffnung blockiert wird und eine Restmenge der Mischung des Zementpulvers (5) mit der Monomerflüssigkeit (4) in einem von dem Hohlzylinder (9) begrenzten Teil des Innenraums der Kartusche (1) verbleibt.

15. Verfahren nach Anspruch 14, **dadurch gekennzeichnet, dass** in Schritt a) die Monomerflüssigkeit (4) durch zumindest eine für das Zementpulver (5) undurchlässige aber für Gase und die Monomerflüssigkeit (4) durchlässige Verbindung (14) im Austragskolben (7) in die Kartusche (1) gepresst wird oder durch die Verbindung (14) und zumindest eine Leitung im Hohlzylinder (9) in die Kartusche (1) gepresst wird, vorzugsweise durch eine Bewegung eines Förderkolbens (6), der mit der Stange (44) der Auspressvorrichtung (43) angetrieben wird, in die Kartusche (1) gepresst wird.

16. Verfahren nach einem der Ansprüche 14 oder 15, **dadurch gekennzeichnet, dass** in Schritt b) durch den auf die Mischung (54) des Zementpulvers (5) mit der Monomerflüssigkeit (4) wirkenden Druck ein Verschluss (36), insbesondere ein Porenfilter (36), in einer Austragsöffnung an der Vorderseite der Kartusche (1) bewegt oder hervorgedrückt wird, wobei bevorzugt hierauf der Verschluss (36) aus der Austragsöffnung entfernt wird und besonders bevorzugt danach ein Applikationsrohr (66) oder eine Austragsrohrverlängerung (70) an der Vorderseite der Kartusche (1) befestigt wird.

## Claims

1. A device for producing a bone cement paste (54) from a monomer liquid (4) and a cement powder (5) as parent components of the bone cement paste (54) and for delivering the mixed bone cement paste (54), the device having a cartridge (1) with a cylindrical interior, wherein the interior of the cartridge (1) is closed at a front side apart from a delivery opening for discharging the bone cement paste (54), wherein a delivery plunger (7) is arranged in the interior of the cartridge (1) the delivery plunger (7) being pushable in the direction of the delivery opening and wherein the cement powder (5) is arranged in the interior of the cartridge (1) between the delivery opening and the delivery plunger (7), wherein
a hollow cylinder (9) is arranged at a front side of the delivery plunger (7) facing the delivery opening, wherein the hollow cylinder (9) is open at a front face facing the delivery opening and extends from the front side of the delivery plunger (7) at least 3 mm into the interior of the cartridge (1), wherein
in the delivery plunger (7) at least one connection (14) is provided from the back side of the delivery plunger (7) to the front side of the delivery plunger (7) for introducing the monomer liquid (4) into the interior of the cartridge (1), wherein the at least one connection (14) is permeable to the monomer liquid (4) and gases and impermeable to the cement powder (5) and wherein the at least one connection (14) leads from the delivery plunger (7) inside the hollow cylinder (9) or through lines in the hollow cylinder (9) at the front face of the hollow cylinder (9) into the interior of the cartridge (1).

2. The device according to Claim 1, **characterized in that**
the hollow cylinder (9) is spaced at its external circumferential surface at most by 0.5 mm from an internal wall of the interior of the cartridge (1), preferably at most 0.1 mm from the internal wall of the interior of the cartridge (1).

3. The device according to Claim 1, **characterized in that**
the hollow cylinder (9) rests at least in places against an internal wall of the interior of the cartridge (1), preferably with its external circumferential surface against the internal wall of the interior of the cartridge (1).

4. The device according to any one of claims 1 to 3, **characterized in that**
the hollow cylinder (9) blocks further movement of the delivery plunger (7) in the direction of a front side of the cartridge (1) when the front face of the hollow cylinder (9) rests against the front side of the interior of the cartridge (1), such that the delivery plunger (7) is spaced from the front side of the interior of the cartridge (1) and a dead volume remains in the interior of the cartridge (1).

5. The device according to any one of the preceding claims, **characterized in that**
the hollow cylinder (9) has at least one slot, preferably at least one slot extending parallel to the cylinder axis of the hollow cylinder (9), particularly preferably at least one slot reaching from the front side to the delivery plunger (7).

6. The device according to any one of the preceding claims, **characterized in that** cement powder (5) is contained, in particular pressed in, in the part of the interior of the cartridge (1) enclosed by the hollow cylinder (9).

7. The device according to any one of the preceding claims, **characterized in that**
the part of the interior of the cartridge (1) defined by the hollow cylinder (9) has a volume of at least 1 cm³ in size, preferably of at least 3 cm³.

8. The device according to any one of the preceding claims, **characterized in that**
the hollow cylinder (9) extends from the front side of the delivery plunger (7) at least 5 mm into the interior of the cartridge (1), preferably at least 7.5 mm into the interior of the cartridge (1), particularly preferably at least 10 mm into the interior of the cartridge (1), and/or
the wall thickness of the hollow cylinder (9) amounts to at least 1 mm, preferably at least 1.5 mm, particularly preferably at least 2 mm.

9. The device according to any one of the preceding claims, **characterized in that**
the device having a receptacle (2) in which the monomer liquid (4), in particular a monomer liquid container (3) containing the monomer liquid (4), is contained, wherein a back side of the cartridge (1) is connected with a front side of the receptacle (2), preferably connected in such a way that the interior of the cartridge (1) is aligned with an interior of the receptacle (2).

10. The device according to Claim 9, **characterized in that**
an interior of the receptacle (2) and the interior of the cartridge (1) are connected together via a connection (14) which is permeable to the monomer liquid (4) and gases but impermeable to the cement powder (5),
the receptacle (2) has a cylindrical interior in which the monomer liquid (4), in particular a monomer liquid container (3) containing the monomer liquid (4), is arranged, a conveying plunger (6) movable in the longitudinal direction of the receptacle (2) is arranged in the receptacle (2), which conveying plunger (6) is advanceable from a back side of the receptacle (2) opposite the front side of the receptacle (2) in the direction of the front side of the receptacle (2), wherein the monomer liquid (4), in particular a monomer liquid container (3) containing the monomer liquid (4), is arranged between the conveying plunger (6) and the delivery plunger (7), and/or
at least one ventilation opening (20), which connects the interior of the receptacle (2) with the surrounding environment, is arranged in a wall of the receptacle (2), wherein the at least one ventilation opening (20) is arranged so close to the conveying plunger (6) such that it is closed by a movement of the conveying plunger (6) in the direction of the front side of the receptacle (2) before a monomer liquid container (3) arranged in the receptacle (2), in which monomer liquid container the monomer liquid (4) is contained, is opened by the movement of the conveying plunger (6).

11. The device according to any one of the preceding claims, **characterized in that**
a fastening means (8) for fastening an expulsion device (43) is arranged on a back side of the device, the delivery plunger (7) being pushable by the fastened expulsion device (43) in the direction of the delivery opening.

12. The device according to any one of the preceding claims, **characterized in that**
the delivery opening is closed at a front side thereof with a closure (36), in particular with a plug (36), wherein the bone cement paste (54) is expellable out of the cartridge (1) through the delivery opening when the delivery opening is open, and wherein the closure (36) is preferably permeable to gases and impermeable to the cement powder (5) and wherein preferably
the closure (36) has an indentation (37) at a back side facing the interior of the cartridge (1), in which indentation the frontmost part of the cement powder (5) is contained.

13. The device according to any one of the preceding claims, **characterized in that**
a cross-section area of the interior of the cartridge (1) amounts to at most 16 cm², preferably at most 5 cm², and/or
the hollow cylinder (9) is formed in one piece with the delivery plunger (7) or with a part of the delivery plunger (7) resting against the internal wall of the cartridge (1).

14. A method for producing a bone cement paste (54), in particular a pasty polymethyl methacrylate bone cement paste (54), wherein the bone cement paste (54) is produced from a cement powder (5) and a monomer liquid (4) using a device according to any one of the preceding claims, having the following succession of steps:
a) inserting the device into an expulsion device (43), the expulsion device (43) having an axially advanceable rod (44),
a1) advancing a conveying plunger (6) with the rod (44) in the direction of the cartridge (1), the conveying plunger (6) being mounted movably inside a receptacle (2) arranged on a back side of the cartridge (1) at the back side of the receptacle (2), wherein through movement of the conveying plunger (6) a monomer liquid container (3), in which the monomer liquid (4) is contained, is opened and pushing the monomer liquid (4) into the interior of the cartridge (1) by the movement of the conveying plunger (6), such that the monomer liquid (4) mixes with the cement powder (5) in the interior of the cartridge (1), wherein the monomer liquid (4) flows around the hollow cylinder (9) before arriving at the internal wall of the cartridge (1),
b) the delivery plunger (7) is advanced with the rod (44) in the direction of the delivery opening of the cartridge (1), wherein the mixture consisting of the cement powder (5) and the monomer liquid (4) from the cartridge (1) is discharged from the device as bone cement paste (54) by the movement of the delivery plunger (7), and
c) the front face of the hollow cylinder (9) meets with the front side of the interior of the cartridge (1), wherein further movement of the delivery plunger (7) in the direction of the delivery opening is blocked with the hollow cylinder (9) and a residual quantity of the mixture of the cement powder (5) with the monomer liquid (4) remains in a part of the interior of the cartridge (1) defined by the hollow cylinder (9).

15. The method according to Claim 14, **characterized in that**
in step a) the monomer liquid (4) is pressed through at least one connection (14) in the delivery plunger (7) impermeable to the cement powder (5) but permeable to gases and the monomer liquid (4) into the cartridge (1) or is pressed through the connection (14) and at least one line in the hollow cylinder (9) into the cartridge (1), preferably is pressed into the cartridge (1) by movement of a conveying plunger (6) which is driven with the rod (44) of the expulsion device (43).

16. The method according to Claim 14 or 15, **characterized in that**
in step b) a closure (36), in particular a porous filter (36), in a delivery opening at the front side of the cartridge (1) is moved or pushed out by the pressure acting on the mixture (54) of the cement powder (5) with the monomer liquid (4), wherein preferably the closure (36) is then removed from the delivery opening and particularly preferably an application tube (66) or a delivery pipe extension (70) is thereafter fastened to the front side of the cartridge (1).

## Revendications

1. Dispositif de fabrication d'une pâte de ciment osseux (54) à partir d'un liquide de monomère (4) et d'une poudre de ciment (5) servant de composants de départ de la pâte de ciment osseux (54), et d'évacuation de la pâte de ciment osseux (54) mélangée, le dispositif présentant
une cartouche (1) avec un espace intérieur cylindrique, dans lequel l'espace intérieur de la cartouche (1) est obturé sur un côté avant jusqu'à un orifice d'évacuation permettant d'expulser la pâte de ciment osseux (54), dans lequel un piston d'évacuation (7) pouvant être poussé en direction de l'orifice d'évacuation est disposé dans l'espace intérieur de la cartouche (1) et dans lequel la poudre de ciment (5) est disposée dans l'espace intérieur de la cartouche (1) entre l'orifice d'évacuation et le piston d'évacuation (7), dans lequel,
un cylindre creux (9) est disposé sur un côté avant du piston d'évacuation (7) orienté vers l'orifice d'évacuation, dans lequel le cylindre creux (9) est ouvert sur son côté avant orienté vers l'orifice d'évacuation et s'étend d'au moins 3 mm dans l'espace intérieur de la cartouche (1), dans lequel au moins une liaison (14), partant du côté arrière du piston d'évacuation (7) jusqu'au côté avant du piston d'évacuation, est prévue dans le piston d'évacuation (7) pour l'approvisionnement du liquide de monomère (4) dans l'espace intérieur de la cartouche (1), dans lequel l'au moins une liaison (14) est perméable pour le liquide de monomère (4) et pour les gaz et ne laisse pas passer la poudre de ciment (5), et dans lequel l'au moins une liaison (14) débouche du piston d'évacuation (7) à l'intérieur du cylindre creux (9), ou débouche par des conduites dans le cylindre creux (9) sur le côté avant du cylindre creux (9) dans l'espace intérieur de la cartouche (1).

2. Dispositif selon la revendication 1, **caractérisé en ce que** le cylindre creux (9) est espacé au niveau de sa surface d'enveloppe extérieure de la paroi intérieure de l'espace intérieur de la cartouche (1) d'au maximum 0,5 mm, de préférence, d'au maximum 0,1 mm de la paroi intérieure de l'espace intérieur de la cartouche (1).

3. Dispositif selon la revendication 1, **caractérisé en ce que**
le cylindre creux (9) est plaqué au moins par endroits contre la paroi intérieure de l'espace intérieur de la cartouche (1), est de préférence plaqué au niveau de sa surface d'enveloppe extérieure contre la paroi intérieure de l'espace intérieur de la cartouche (1).

4. Dispositif selon l'une des revendications précédentes, **caractérisé en ce que** le cylindre creux (9) bloque un nouveau déplacement du piston d'évacuation (7) en direction du côté avant de la cartouche (1) lorsque le côté avant du cylindre creux (9) est plaqué contre le côté avant de l'espace intérieur de la cartouche (1), de sorte que le piston d'évacuation (7) est espacé par rapport au côté avant de l'espace intérieur de la cartouche (1) et qu'il reste un volume mort dans l'espace intérieur de la cartouche (1).

5. Dispositif selon l'une des revendications précédentes, **caractérisé en ce que** le cylindre creux (9) présente au moins une fente, de préférence, présente au moins une fente s'étendant parallèlement par rapport à l'axe de cylindre du cylindre creux (9), de manière particulièrement préférée, présente au moins une fente allant du côté avant jusqu'au piston d'évacuation (7).

6. Dispositif selon l'une des revendications précédentes, **caractérisé en ce que** de la poudre de ciment (5) est contenue dans la partie de l'espace intérieur de la cartouche (1) entourée par le cylindre creux (9), en particulier, est pressée à l'intérieur.

7. Dispositif selon l'une des revendications précédentes, **caractérisé en ce que** le volume de l'espace intérieur de la cartouche (1) délimité par le cylindre creux (9) est d'une taille d'au moins 1 cm³, de préférence d'au moins 3 cm³.

8. Dispositif selon l'une des revendications précédentes, **caractérisé en ce que** le cylindre creux (9) s'étend à partir du côté avant du piston d'évacuation (7) d'au moins 5 mm dans l'espace intérieur de la cartouche (1), s'étend de préférence d'au moins 7,5 mm dans l'espace intérieur de la cartouche (1), s'étend de manière particulièrement préférée d'au moins 10 mm dans l'espace intérieur de la cartouche (1), et/ou
l'épaisseur de la paroi du cylindre creux (9) est d'au moins 1 mm, est de préférence d'au moins 1,5 mm, est de manière particulièrement préférée d'au moins 2 mm.

9. Dispositif selon l'une des revendications précédentes, **caractérisé en ce que** le dispositif présente un réceptacle (2) dans lequel le liquide de monomère (4), notamment un récipient de liquide de monomère (3) contenant le liquide de monomère (4), est contenu, dans lequel le côté arrière de la cartouche (1) est relié avec le côté avant du réceptacle (2), de préférence relié de telle manière que l'espace intérieur de la cartouche (1) est aligné avec l'espace intérieur du réceptacle (2).

10. Dispositif selon la revendication 9, **caractérisé en ce**
**qu'**un espace intérieur du réceptacle (2) et que l'espace intérieur de la cartouche (1) sont reliés ensemble par le biais d'une liaison (14) perméable pour le liquide de monomère (4) et pour les gaz, mais ne laissant pas passer la poudre de ciment (5),
**que** le réceptacle (2) présente un espace intérieur cylindrique dans lequel le liquide de monomère (4), notamment un récipient de liquide de monomère (3) contenant le liquide de monomère (4), est disposé,
**qu'**un piston de transport (6) mobile dans la direction longitudinale du réceptacle (2) est disposé dans le réceptacle (2), qui peut être avancé en direction du côté avant par le côté arrière du réceptacle (2), dans lequel le liquide de monomère (4), notamment un récipient de liquide de monomère (3) contenant le liquide de monomère (4), est disposé entre le piston de transport (6) et le piston d'évacuation (7), et/ou
**qu'**au moins un orifice d'aération (20) est disposé dans la paroi du réceptacle (2), qui relie l'espace intérieur du réceptacle (2) avec l'environnement, dans lequel l'au moins un orifice d'aération (20) est disposé tellement près de la zone du piston de transport (6) qu'il s'obture par un déplacement du piston de transport (6) en direction du côté avant du réceptacle (2) avant qu'un récipient de liquide de monomère (3) disposé dans le réceptacle, dans lequel est contenu le liquide de monomère (4), soit ouvert par le déplacement du piston de transport (6).

11. Dispositif selon l'une des revendications précédentes, **caractérisé en ce qu'**un système de fixation (8) est disposé dans le coté arrière du dispositif, pour la fixation d'un dispositif d'expulsion par pressage (43) avec lequel le piston d'évacuation (7) peut être pressé en direction de l'orifice d'évacuation.

12. Dispositif selon l'une des revendications précédentes, **caractérisé en ce que** l'orifice d'évacuation est fermé au niveau de son côté avant avec un dispositif de fermeture (36), notamment avec un bouchon (36), dans lequel la pâte de ciment osseux (54) peut être pressée hors de la cartouche (1) par l'orifice d'évacuation lorsque l'orifice d'évacuation est ouvert, et dans lequel, de préférence, le système de fermeture (36) est perméable pour les gaz et ne laisse pas passer la poudre de ciment (5), et dans lequel, de préférence,
le système de fermeture (36) présente, dans le côté arrière orienté vers l'espace intérieur de la cartouche (1), une cavité (37) dans laquelle la partie la plus en avant de la poudre de ciment (5) est contenue.

13. Dispositif selon l'une des revendications précédentes, **caractérisé en ce que** la section transversale de l'espace intérieur de la cartouche (1) est d'au maximum 16 cm², de préférence au maximum de 5 cm², et/ou le cylindre creux (9) est conçu en une seule pièce avec le piston d'évacuation (7) ou est formé avec une partie du piston d'évacuation (7) adjacente à la paroi intérieure de la cartouche (1).

14. Procédé de fabrication d'une pâte de ciment osseux (54), notamment d'une pâte de ciment en poly méthyl méthacrylate (54) en forme de pâte, dans lequel la pâte de ciment osseux (54) est fabriquée à partir d'une poudre de ciment (5) et d'un liquide de monomère (4) avec un dispositif selon l'une des revendications précédentes, avec les étapes suivantes se déroulant l'une après l'autre :
a) l'emploi du dispositif dans un dispositif d'expulsion par pressage (43), le dispositif d'expulsion par pressage (43) présentant une tige (44) pouvant avancer axialement,
a1) l'avancée d'un piston de transport (6) avec la tige (44) en direction de la cartouche (1), qui est logé en étant mobile sur le côté arrière du réceptacle (2), à l'intérieur du réceptacle (2) disposée sur le côté arrière de la cartouche (1), dans lequel, par le déplacement du piston de transport (6), un récipient de liquide de monomère (3), dans lequel est contenu le liquide de monomère (4), est ouvert, et le pressage du liquide de monomère (4) dans l'espace intérieur de la cartouche (1) par le déplacement du piston de transport (6) de sorte que le liquide de monomère (4) se mélange à la poudre de ciment (5) dans l'espace intérieur de la cartouche (1), dans lequel le liquide de monomère (4) s'écoule autour du cylindre creux (9) avant qu'il n'atteigne la paroi intérieure de la cartouche (1),
b) le piston d'évacuation (7) est avancé avec la tige (44) en direction de l'orifice d'évacuation de la cartouche (1), dans lequel, par le déplacement du piston d'évacuation (7), le mélange à base de poudre de ciment (5) et de liquide de monomère (4) est expulsé hors de la cartouche (1) sous forme de pâte de ciment osseux (64) hors du dispositif, et
c) le côté avant du cylindre creux (9) rencontre le côté avant de l'espace intérieur de la cartouche (1), dans lequel un nouveau déplacement du piston d'évacuation (7) avec le cylindre creux (9) est bloqué en direction de l'orifice d'évacuation et une quantité résiduelle du mélange de la poudre de ciment (5) avec le liquide de monomère (4) reste dans une partie de l'espace intérieur de la cartouche (1) délimitée par le cylindre creux (9).

15. Procédé selon la revendication 14, **caractérisé en ce que** dans l'étape a), le liquide de monomère (4) est pressé à travers au moins une liaison (14), ne laissant pas passer la poudre de ciment (5) mais perméable pour les gaz et le liquide de monomère (4), dans le piston d'évacuation (7) dans la cartouche (1) ou est pressé à travers la liaison (14) et au moins une conduite dans le cylindre creux (9) dans la cartouche (1), est pressé dans la cartouche (1) de préférence par un déplacement d'un piston de transport (6) qui est entraîné avec la tige (44) du dispositif d'expulsion par pressage (43).

16. Procédé selon l'une des revendications 14 ou 15, **caractérisé en ce que** dans l'étape b), un système de fermeture (36), notamment un filtre poreux (36), est déplacé ou avancé dans un orifice d'évacuation sur le côté avant de la cartouche (1) par la pression agissant sur le mélange (54) de la poudre de ciment (5) avec le liquide de monomère (4), dans lequel, de préférence, suite à cela, le système de fermeture (36) est retiré de l'orifice d'évacuation et de manière particulièrement préférée, ensuite un tuyau d'application (66), ou un prolongement de tuyau d'évacuation (70), est fixé sur le côté avant de la cartouche (1).
